# EUROPEAN PATENT SPECIFICATION

(11) **EP 2 281 065 B1**
(45) Date of publication and mention of the grant of the patent: **09.09.2015**
(21) Application number: 09743242.1
(22) Date of filing: 16.04.2009
(51) Int. Cl.: C12Q 1/68

(54) **METHOD FOR EVALUATING AND COMPARING IMMUNOREPERTOIRES**
VERFAHREN FÜR DIE BEURTEILUNG UND DEN VERGLEICH VON IMMUNREPERTOIRES
METHODE D EVALUATION ET DE COMPARAISON D IMMUNO-REPERTOIRES

(30) Priority: 16.04.2008 US 45586 P
(43) Date of publication of application: 09.02.2011
(73) Proprietor: HudsonAlpha Institute For Biotechnology, Huntsville, AL 35806 (US)
(72) Inventor: HAN, Jian, Huntsville,AL 35802 (US)
(74) Representative: Webber, Philip Michael
(86) International application number: PCT/US2009/040855
(87) International publication number: WO 2009/137255

(56) References cited:
- EP-A1- 1 544 308
- WO-A2-99/19509
- WO-A2-2004/005465
- WO-A2-2004/033728
- WO-A2-2004/063706
- WO-A2-2005/038039
- US-A1- 2004 077 003
- US-A1- 2006 129 331
- US-A1- 2007 117 134
- CORONELLA J A ET AL: "Amplification of IgG VH and VL (Fab) from single human plasma cells and B cells", NUCLEIC ACIDS RESEARCH, OXFORD UNIVERSITY PRESS, SURREY, GB, vol. 28, no. 20, 15 October 2000 (2000-10-15), pages E85-1, XP002533174, ISSN: 0305-1048, DOI: DOI:10.1093/NAR/28.20.E85
- VAN DONGEN J ET AL: "Design and standardization of PCR primers and protocols for detection of clonal immunoglobuline and T-cell receptor gene recombinations is suspect lymphoproliferations: report of the BIOMED-2 concerted action BMH4-CT98-3936", LEUKEMIA, MACMILLAN PRESS LTD, US, vol. 17, no. 12, 1 December 2000 (2000-12-01), pages 2257-2317, XP008093070, ISSN: 0887-6924
- HEATH K E ET AL: "Universal Primer Quantitative Fluorescent Multiplex (UPQFM) PCR: A Method To Detect Major And Minor Rearrangements Of The Low Density Lipoprotein Receptor Gene", JOURNAL OF MEDICAL GENETICS, BMJ PUBLISHING GROUP, LONDON, GB, vol. 37, no. 4, 1 April 2000 (2000-04-01), pages 272-280, XP001055883, ISSN: 0022-2593, DOI: DOI:10.1136/JMG.37.4.272
- LIN Z ET AL: "Multiplex genotype determination at a large number of gene loci", PROCEEDINGS OF THE NATIONAL ACADEMY OF SCIENCES, NATIONAL ACADEMY OF SCIENCES, WASHINGTON, DC; US, vol. 93, no. 6, 19 March 1996 (1996-03-19) , pages 2582-2587, XP002142667, ISSN: 0027-8424, DOI: DOI:10.1073/PNAS.93.6.2582
- DATABASE Geneseq [Online] 7 April 2005 (2005-04-07), "TCR beta chain variable region BV5 gene forward PCR primer, SEQ ID 16.", XP002651258, retrieved from EBI accession no. GSN:ADW05236 Database accession no. ADW05236 & US 2005/010030 A1 (ZANG JINGWU Z [US] ET AL) 13 January 2005 (2005-01-13)
- DATABASE Geneseq [Online] 31 January 2001 (2001-01-31), "TCR Valpha 11 subfamily probe VA11-1.", XP002651259, retrieved from EBI accession no. GSN:AAA95563 Database accession no. AAA95563 & JP 2000 157297 A (SHIONOGI & CO) 13 June 2000 (2000-06-13)
- DATABASE EMBL [Online] 21 January 2000 (2000-01-21), "Sequence 18 from Patent WO9927957.", XP002651260, retrieved from EBI accession no. EM_PAT:A80530 Database accession no. A80530 & WO 99/27957 A1 (IMMUNE RESPONSE CORP INC [US]; SIDNEY KIMMEL CANCER CT [US]) 10 June 1999 (1999-06-10)
- E BERTOLINI: "Highly sensitive detection of Pseudomonas savastanoi pv. savastanoi in asymptomatic olive plants by nested-PCR in a single closed tube", JOURNAL OF MICROBIOLOGICAL METHODS, vol. 52, no. 2, 1 February 2003 (2003-02-01), pages 261-266, XP55002901, ISSN: 0167-7012, DOI: 10.1016/S0167-7012(02)00163-X
- PRINZ ET AL.: 'Selection of conserved TCR VDJ rearrangements in chronic psoriatic plaques indicates a common antigen in psoriasis vulgaris' EUR. J. IMMUN. vol. 29, no. 10, October 1999, pages 3360 - 3368, XP008145210
- C. Wang ET AL: "High throughput sequencing reveals a complex pattern of dynamic interrelationships among human T cell subsets", Proceedings of the National Academy of Sciences, vol. 107, no. 4, 4 January 2010 (2010-01-04), pages 1518-1523, XP055114155, ISSN: 0027-8424, DOI: 10.1073/pnas.0913939107
- H. S. Robins ET AL: "Comprehensive assessment of T-cell receptor -chain diversity in T cells", Blood, vol. 114, no. 19, 5 November 2009 (2009-11-05), pages 4099-4107, XP055074444, ISSN: 0006-4971, DOI: 10.1182/blood-2009-04-217604
- Heger, M: "Studies Highlight Challenges of Immune Repertoire Sequencing's Clinical Applicability", , 22 March 2011 (2011-03-22), Retrieved from the Internet: URL:http://www.genomeweb.com/print/964535 [retrieved on 2012-06-19]
- Frost & Sullivan Press Release: "Frost & Sullivan Recognizes iCubate for developing the New Gold Standard Instrument in the PCR Market", , 22 March 2012 (2012-03-22), Retrieved from the Internet: URL:http://www.frost.com/prod/servlet/pres s-release-print.pag?docid=256367538 [retrieved on 2012-06-16]

## Description

### Field of the Invention

The invention relates to methods for producing an immune status profile for a human and/or animal.

### Background of the Invention

Scientists have known for a number of years that certain diseases are associated with particular genes or genetic mutations. Genetic causation, however, accounts for only a portion of the diseases diagnosed in humans. Many diseases appear to be linked in some way to the immune system's response to infectious and environmental agents, but how the immune system plays a role in diseases such as cancer, Alzheimer's, costochondritis, fibromyalgia, lupus, and other diseases is still being determined.

The human genome comprises a total number of 567-588 Ig (immunoglobulin) and TR (T cell receptor) genes (339-354 Ig and 228-234 TR) per haploid genome, localized in the 7 major loci. They comprise 405-418 V, 32 D, 105-109 J and 25-29 C genes. The number of functional Ig and TR genes is 321-353 per haploid genome. They comprise 187-216 V, 28 D, 86-88 J and 20-21 C genes (http://imgt.cines.fr). Through rearrangement of these genes, it has been estimated that approximately 2.5x10⁷ possible antibodies or T cell receptors can be generated.

Although, at the germline level, human beings are capable of generating large numbers of diverse Igs and TRs, the number of available Igs and TRs for a particular individual is actually much smaller due to negative selection during B and T cell development. In some individuals, this process may not remove some of the cells that would cross-react with the body's own tissues, and this may be the cause of some types of autoimmune diseases.

A few diseases to date have been associated with the body's reaction to a common antigen (Prinz, J. et al., Eur. J. Immunol. (1999) 29(10): 3360-3368, "Selection of Conserved TCR VDJ Rearrangements in Chronic Psoriatic Plaques Indicates a Common Antigen in Psoriasis Vulgaris") and/or to specific VDJ rearrangements (Tamaru, J. et al., Blood (1994) 84(3): 708-715, "Hodgkin's Disease with a B-cell Phenotype Often Shows a VDJ Rearrangement and Somatic Mutations in the VH Genes"). Coronella et al. (Nucleic Acids Research 2000, vol 28(20), p. e85) discloses the amplification of IgG VH and VL (Fab) from single human plasma cells and B cells. What is needed is a better method for evaluating changes in human immune response cells and associating those changes with specific diseases.

### Summary of the Invention

The invention relates to a method for producing an immune status profile (ISP) for a human and/or animal. In one aspect of the invention, the method comprises the steps of: (a) amplifying, in a first multiplex PCR amplification using nested target-specific primers, multiple DNAs and/or RNAs from a sample of white blood cells from a human or animal subject to produce multiple first amplicons, at least a portion of the inner target-specific primers comprising additional nucleotides to incorporate into the first amplicons a binding site for a common primer; (b) separating the amplicons from the target-specific primers or diluting the amplicon/target specific primer mix so that there are significantly more amplicons than target-specific primers from the first amplification; (c) amplifying, in a second amplification using at least one common primer, the first amplicons to produce multiple second amplicons; and (d) sequencing the second amplicons to identify and quantify DNA sequences representing rearrangements to create an immune status profile.

Genomic DNA may also be amplified, and the step of amplifying DNA may be substituted for the step of amplifying RNA, especially in cases where analysis of an immune system component such as the major histocompatibility complex (MHC) is desired.

In some preferred aspects of the method of the invention, subpopulations of white blood cells may be isolated by flow cytometry to separate naive B cells, mature B cells, memory B cells, naïve T cells, mature T cells, and memory T cells. In various aspects of the method, recombinations in the subpopulation of cells are rearrangements of B-cell immunoglobulin heavy chain (IgH), kappa and/or lambda light chains (IgK, IgL), T-cell receptor Beta, Gamma, Delta, and/or Major Histocompatibility Complex (MHC) molecules I or II.

In another aspect of the invention, the method may also comprise compiling and comparing the immune cell profile for a population of normal individuals with the immune cell profile for a population of individuals who have been diagnosed with a disease to determine if there is a correlation between a specific rearrangement or set of rearrangements and the disease.

Also disclosed is a method which comprises comparing the immune cell profile identified for a population of individuals to whom a vaccine has been administered with the immune cell profile for a population of individuals to whom the vaccine was not administered to evaluate the efficacy of the vaccine in producing an immune response.

### Brief Description of the Drawings

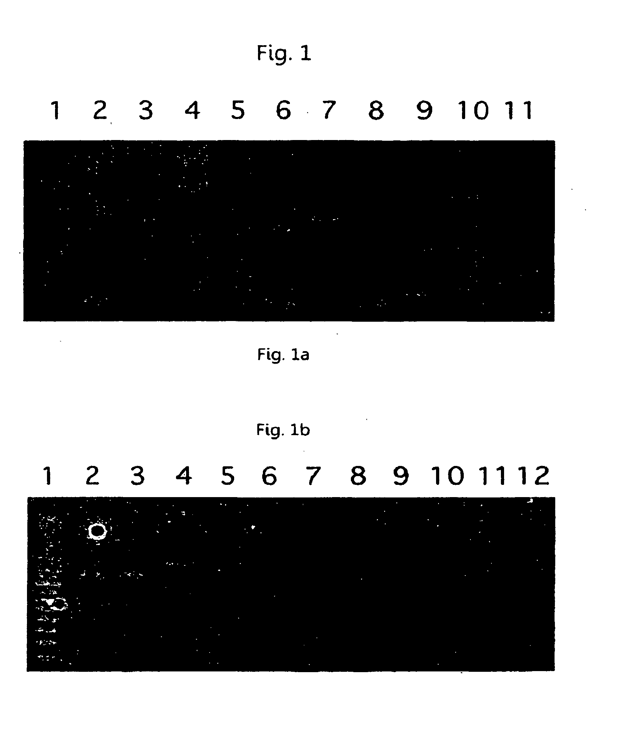

Figure 1a and Figure 1b are photographs of gels illustrating the presence of amplification products obtained by the method of the invention using primers disclosed herein.

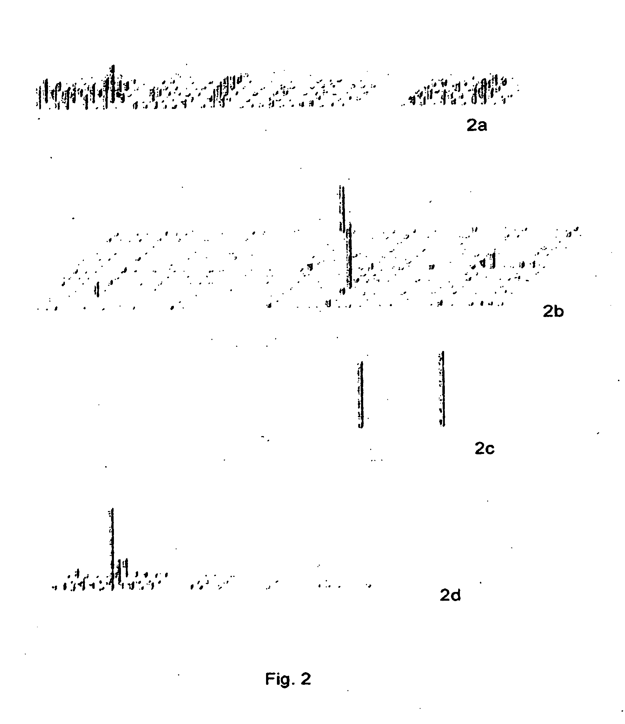

Figure 2 illustrates distributions of domain usage for (a) Ig heavy chain in healthy control sample, (b) TCR beta chain in a blood sample from a patient with colon cancer, (c) Ig kappa chain in a blood sample from a patient with Chronic Lymphocytic Leukemia (CLL), and (d) Ig lambda chain in a blood sample from a patient with Systemic lupus erythematosus (SLE). Empty spots indicate missing sequences associated with corresponding V-J combinations and the height of the column indicates the frequency of occurrence of a particular sequence.

### Detailed Description

The inventor has developed a method for evaluating antibody and receptor rearrangements from a large number of cells, the method being useful for comparing rearrangements identified in populations of individuals to determine whether there is a correlation between a specific rearrangement or set of rearrangements and a disease, or certain symptoms of a disease. The method is also useful for establishing a history of the immune response of an individual or individuals in response to infectious and/or environmental agents, as well as for evaluating the efficacy of vaccines.

The invention relates to a method for producing an immune status profile (ISP) for a human and/or animal, the method comprising the steps of (a) amplifying, in a first multiplex PCR amplification using nested target-specific primers, multiple DNAs and/or RNAs from a sample of white blood cells from a human or animal subject to produce multiple first amplicons, at least a portion of the inner target-specific primers comprising additional nucleotides to incorporate into the first amplicons a binding site for a common primer; (b) separating the amplicons from the target-specific primers or diluting the amplicon/target specific primer mix so that there are significantly more amplicons than target-specific primers from the first amplification; (c) amplifying, in a second amplification using at least one common primer, the first amplicons to produce multiple second amplicons; and (d) sequencing the second amplicons to identify and quantify DNA sequences representing rearrangements to create an immune status profile.

Where the term "comprising" is used herein, "consisting essentially of" and "consisting of" may also be used. The term "immune status profile" is intended to mean a profile for an individual or population of individuals indicating the presence and/or absence of sequences representing specific rearrangements representing the diversity of B cells, T cells, and/or other cells of the human and/or animal immune system, as well as the frequency of their occurrence. Where amplicons are referred to as "rescued" herein, it is to be understood that amplicon rescue may occur by the separation of amplicons from the primers which are used to create them, or may occur by dilution of the amplicon/primer mix so that, by virtue of the fact that there are significantly more amplicons than primers from a first amplification reaction, the effect of those primers is minimized in a second amplification reaction using different primers. "Common primers" are those primers that may be used to amplify polynucleotides (e.g., amplicons from a first amplification produced by target-specific primers) having non-identical sequences in general, but sharing sequence similarities in that they contain binding sites for the same primers. Common primers are generally chosen for their efficiency at priming successful amplifications, so their use is effective for achieving higher levels of amplification in a non-target-specific manner in the method of the present invention. Common primer binding sites may be incorporated into amplicons resulting from a first amplification by attaching their sequences or their complementary sequences to the sequence of a target-specific primer. Common primers may be chosen by one of skill in the art by a variety of primer-design methods.

Subpopulations of white blood cells may be isolated by flow cytometry to separate naïve B cells, mature B cells, memory B cells, naïve T cells, mature T cells, and memory T cells. Recombinations in these subpopulations of cells are generally rearrangements of B-cell immunoglobulin heavy chain (IgH), kappa and/or lambda light chains (IgK, IgL), T-cell receptor Beta, Gamma, Delta, and/or Major Histocompatibility Complex (MHC) molecules I or II.

By performing an additional step, namely that of compiling and comparing the average immune status profile for a population of normal individuals with an average immune status profile for a population of individuals who have been diagnosed with a disease, it is possible to use the immune cell profile to determine if there is a correlation between a specific rearrangement or set of rearrangements and the disease.

Also disclosed herein is a method for evaluating vaccine efficacy, in terms of creating a change in the immune cell profile, by performing the steps of the method and comparing the immune cell profile identified for a population of individuals to whom a vaccine has been administered with the immune cell profile for a population of individuals to whom the vaccine was not administered to evaluate the efficacy of the vaccine in producing an immune response.

A peripheral blood sample may be taken from a patient and isolation of a subpopulation of white blood cells may be performed by flow cytometry to separate naïve B cells, mature B cells, memory B cells, naïve T cells, mature T cells, and memory T cells. In various embodiments of the method, the recombinations in the subpopulation of cells comprise rearrangements of B-cell immunoglobulin heavy chain (IgH), kappa and/or lambda light chains (IgK, IgL), T-cell receptor Beta, Gamma, Delta, or Major Histocompatibility Complex (MHC) molecules I or II.

The inventor previously developed a PCR method known as tem-PCR, which has been described in publication number WO2005/038039. More recently, the inventor has developed a method called amplicon rescue multiplex polymerase chain reaction (arm-PCR), which is described in WO2009/124293 and herein. Both the tem-PCR and arm-PCR methods provide semi-quantitative amplification of multiple polynucleotides in one reaction. Additionally, arm-PCR provides added sensitivity. Both provide the ability to amplify multiple polynucleotides in one reaction, which is beneficial in the present method because the repertoire of various T and B cells, for example, is so large. The addition of a common primer binding site in the amplification reaction, and the subsequent amplification of target molecules using common primers, gives a quantitative, or semi-quantitative result-making it possible to determine the relative amounts of the cells comprising various rearrangements within a patient blood sample. Clonal expansion due to recognition of antigen results in a larger population of cells which recognize that antigen, and evaluating cells by their relative numbers provides a method for determining whether an antigen exposure has influenced expansion of antibody-producing B cells or receptor-bearing T cells. This is helpful for evaluating whether there may be a particular population of cells that is prevalent in individuals who have been diagnosed with a particular disease, for example, and may be especially helpful in evaluating whether or not a vaccine has achieved the desired immune response in individuals to whom the vaccine has been given.

There are several commercially available high throughput sequencing technologies, such as Roche Life Sciences 454 Sequencing^{®}. In this sequencing method, 454A and 454B primers are either linked onto PCR products during PCR or ligated on after the PCR reaction. When done in conjunction with tem-PCR or arm-PCR, 454A and 454B primers may be used as common primers in the amplification reactions. PCR products, usually a mixture of different sequences, are diluted to about 200 copies per pl. In an "emulsion PCR" reaction, (a semisolid gel like environment) the diluted PCR products are amplified by primers (454A or 454B) on the surface of the microbeads. Because the PCR templates are so dilute, usually only one bead is adjacent to one template, and confined in the semisolid environment, amplification only occurs on and around the beads. The beads are then eluted and put onto a plate with specially designed wells. Each well can only hold one bead. Reagents are then added into the wells to carry out pyrosequencing. A fiber-optic detector may be used to read the sequencing reaction from each well and the data is collected in parallel by a computer. One such high throughput reaction could generate up to 60 million reads (60 million beads) and each read can generate about 300bp sequences.

One aspect of the present disclosure involves the development of a database of immune status profiles, or "personal immunorepertoires" (PIRs), so that each individual may establish a baseline and follow the development of immune responses to antigens, both known and unknown, over a period of years. This information may, if information is gathered from a large number of individuals, provide an epidemiological database that will produce valuable information, particularly in regard to the development of those diseases such as cancer and heart disease which are thought to often arise from exposure to viral or other infectious agents, many of which have as yet been unidentified. One particularly important use for the method of the invention enables studies of children to determine whether infectious disease, environmental agents, or vaccines may be the cause of autism. For example, many have postulated that vaccine administration may trigger the development of autism. However, many also attribute that potential correlation to the use of agents such as thimerosol in the vaccine, and studies have demonstrated that thimerosol does not appear to be a causative agent of the disease. There is still speculation that the development of cocktail vaccines has correlated with the rise in the number of cases of autism, however, but gathering data to evaluate a potential causal connection for multiple antigens is extremely difficult. The method of the present invention simplifies that process and may provide key information for a better understanding of autism and other diseases in which the immune response of different individuals may provide an explanation for the differential development of disease in some individuals exposed to an agent or a group of agents, while others similarly exposed do not develop the disease.

Imbalances of the PIR, triggered by infection, may lead to many diseases, including cancers, leukemia, neuronal diseases (Alzheimer's, Multiple Sclerosis, Parkinson's, autism etc), autoimmune diseases, and metabolic diseases. These diseases may be called PIR diseases. There may be two PIR disease forms. (1) a "loss of function" form, and (2) a "gain of function" form. In the "loss of function" form, a person is susceptible to a disease because his/her restricted and/or limited PIR lacks the cells that produce the most efficient and necessary Igs and TRs. In the "gain of function" form, a person is susceptible to a disease because his/her PIR gained cells that produce Igs and TRs that normally should not be there. In the "loss of function" (LOF) PIR diseases, an individual does not have the appropriate functional B or T cells to fight a disease. His/her HLA typing determines that those cells are eliminated during the early stages of the immune cell maturation process, the cells generally being eliminated because they react to strongly to his/her own proteins.

One preferred aspect of the method of the invention additionally comprises entering a patient immune cell profile into a database in combination with identifying information such as, for example, a patient identification number, a code comprising the patient's HLA type, a disease code comprising one or more clinical diagnoses that may have been made, a "staging code" comprising the date of the sample, a cell type code comprising the type of cell subpopulation from which the RNA was amplified and sequenced, and one or more sequence codes comprising the sequences identified for the sample.

The described method includes a primer set that not only allows amplification of the entire immunorepertoire, but also allows multiplex amplification that is semi-quantitative. Multiplex amplification requires that only a few PCR or RT-PCR reactions are needed. For example, all immunoglobulin (Ig) sequences present may be amplified in one reaction, or two or three reactions may be peformed separately, using primers specific for IgH, IgL or IgK. Similarly, the T-cell receptors (TRs) may be amplified in just one reaction, or may be amplified in a few reactions including the T-cell receptors designated TRA, TRB, TRD, and TRG. MHC genes may be amplified in just one PCR reaction. Semi-quantitative amplification allows all the targets in the multiplex reaction to be amplified independently, so that the end point analysis of the amplified products will reflect the original internal ratio among the targets. Because this ratio is maintained, it is possible to produce an immune cell profile that indicates the presence or absence, as well as relative numbers, of various immune system cells. Amplification of RNA according to the method of the invention may be performed using any or all of the primers listed in Tables 1, 2, and/or 3. The invention therefore provides a method according to claim 1 wherein the target-specific primers are chosen from among the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126. SEQ ID NO: 127, SEQ ID NO: 128, SEQ ID NO: 129, SEQ ID NO: 130, SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID N0:138, SEQ ID NO: 139, SEQ ID NO: 140, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, SEQ ID NO: 162, SEQ ID NO: 163, SEQ ID NO: 164, SEQ ID NO: 165, SEQ ID NO: 166, SEQ ID NO: 167, SEQ ID NO: 168, SEQ ID NO: 169, SEQ ID NO: 170, SEQ ID NO: 171, SEQ ID NO: 172, SEQ ID NO: 173, SEQ ID NO: 174, SEQ ID NO: 175, SEQ ID NO: 176, SEQ ID NO: 177, SEQ ID NO: 178, SEQ ID NO: 179, SEQ ID NO: 180, SEQ ID NO: 181, SEQ ID NO: 182, SEQ ID NO: 183, SEQ ID NO: 184, SEQ ID NO: 185, SEQ ID NO: 186, SEQ ID NO: 187, SEQ ID NO: 188, SEQ ID NO: 189, SEQ ID NO: 190, SEQ ID NO: 191, SEQ ID NO: 192, SEQ ID NO: 193, SEQ ID NO: 194, SEQ ID NO: 195, SEQ ID NO: 196, SEQ ID NO: 197, SEQ ID NO: 198, SEQ ID NO: 199, SEQ ID NO: 200, SEQ ID NO: 201, SEQ ID NO: 202, SEQ ID NO: 203, SEQ ID NO: 204, SEQ ID NO: 205, SEQ ID NO: 206, SEQ ID NO: 207, SEQ ID NO: 208, SEQ ID NO: 209, SEQ ID NO: 210, SEQ ID NO: 211, SEQ ID NO: 212, SEQ ID NO: 213, SEQ ID NO: 214, SEQ ID NO: 215, SEQ ID NO: 216, SEQ ID NO: 217, SEQ ID NO: 218, SEQ ID NO: 219, SEQ ID NO: 220, SEQ ID NO: 221, SEQ ID NO: 222, SEQ ID NO: 223, SEQ ID NO: 224, SEQ ID NO: 225, SEQ ID NO: 226, SEQ ID NO: 227, SEQ ID NO: 228, SEQ ID NO: 229, SEQ ID NO: 230, SEQ ID NO: 231, SEQ ID NO: 232, SEQ ID NO: 233, SEQ ID NO: 234, SEQ ID NO: 235, SEQ ID NO: 236, SEQ ID NO: 237, SEQ ID NO: 238, SEQ ID NO: 239, SEQ ID NO: 240, SEQ ID NO: 241, SEQ ID NO: 242, SEQ ID NO: 243, SEQ ID NO: 244, SEQ ID NO: 245, SEQ ID NO: 246, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255, SEQ ID NO: 256, SEQ ID NO: 257, SEQ ID NO: 258, SEQ ID NO: 259, SEQ ID NO: 260, SEQ ID NO: 261, SEQ ID NO: 262, SEQ ID NO: 263, SEQ ID NO: 264, SEQ ID NO: 265, SEQ ID NO: 266, SEQ ID NO: 267, SEQ ID NO: 268, SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, SEQ ID NO: 281, SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289, SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, SEQ ID NO: 300, SEQ ID NO: 301, SEQ ID NO: 302, SEQ ID NO: 303, SEQ ID NO: 304, SEQ ID NO: 305, SEQ ID NO: 306, SEQ ID NO: 307, SEQ ID NO: 308, SEQ ID NO: 309, SEQ ID NO: 310, SEQ ID NO: 311, and SEQ ID NO: 312.
The listed primers were designed by the inventor to provide efficient amplification of their respective RNA and/or DNA targets. Use of the entire group of primers is effective to produce a detailed immune status profile for an individual. Use of a subset may, however, be desired when specific populations of T or B cells, for example, are the subject of particular interest.

By way of further explanation, the following example may be illustrative of the methods of the invention. Blood samples may be taken from children prior to administration of any vaccines, those blood samples for each child being used in the method of the invention to create a "baseline" immune cell profile or personal immunorepertoire (PIR) from which future immune cell profiles, created from blood samples taken during later years and analyzed by the method of the invention, may be compared. For each child, the future samples may be utilized to determine whether there has been an exposure to an agent which has expanded a population of cells known to be correlated with a disease, and this may serve as a "marker" for the risk of development of the disease in the future. Individuals so identified may then be more closely monitored so that early detection is possible, and any available treatment options may be provided at an earlier stage in the disease process.

The method of the invention may be especially useful for identifying commonalities between individuals with autoimmune diseases, for example, and may provide epidemiological data that will better describe the correlation between infectious and environmental factors and diseases such as heart disease, atherosclerosis, diabetes, and cancer providing biomarkers that signal either the presence of a disease, or the tendency to develop disease.

The method may also be useful for development of passive immunity therapies. For example, following exposure to an infectious agent, certain antibody-producing B cells and/or T cells are expanded. The method of the invention enables the identification of protective antibodies, for example, and those antibodies may be utilized to provide passive immunity therapies in situations where such therapy is needed.

The method of the invention may also provide the ability to accomplish targeted removal of cells with undesirable rearrangements, the method providing a means by which such cells rearrangements may be identified.

The inventor has identified and developed target-specific primers for use in the method of the invention. T-cell-specific primers are shown in Table 1, antibody-specific primers are shown in Table 2, and HLA-specific primers are shown in Table 3. Therefore, the method may comprise using any combination of primers of Table 1, Table 2, and/or Table 3 to amplify RNA and/or DNA from a blood sample, and more particularly to identify antibodies, T-cell receptors, and HLA molecules within a population of cells. For example, an analysis of T-cell distribution might utilize all or a portion of the primers listed in Table 1 (SEQ ID NO: 1 through SEQ ID NO: 157). An analysis of Ig might utilize all or a portion of the primers listed in Table 2 (SEQ ID NO: 158 through SEQ ID NO: 225), and an analysis of HLA distribution might utilize all or a portion of the primers listed in Table 3 (SEQ ID NO: 159 through SEQ ID NO: 312).

In a tem-PCR reaction, nested gene-specific primers are designed to enrich the targets during initial PCR cycling. Later, universal "Super" primers are used to amplify all targets. Primers are designated as Fₒ (forward out), Fᵢ (forward in), Rᵢ (reverse in), Rₒ₍reverse out), FS (forward super primer) and RS,(reverse super primer), with super primers being common to a variety of the molecules due to the addition of a binding site for those primers at the end of a target-specific primer. The gene-specific primers (Fₒ, Fᵢ, Rᵢ, and Rₒ) are used at extremely low concentrations. Different primers are involved in the tem-PCR process at each of the three major stages. First, at the "enrichment" stage, low-concentration gene-specific primers are given enough time to find the templates. For each intended target, depending on which primers are used, four possible products may be generated: Fₒ/Rₒ, Fᵢ/Rₒ, Fᵢ/Rᵢ, and Fₒ/Rᵢ. The enrichment stage is typically carried out for 10 cycles. In the second, or "tagging" stage, the annealing temperature is raised to 72°C, and only the long 40-nucleotide inside primers (Fi and Ri) will work. After 10 cycles of this tagging stage, all PCR products are "tagged" with the universal super primer sequences. Then, at the third "amplification" stage, high-concentration super primers work efficiently to amplify all targets and label the pCR products with biotin during the process. Specific probes may be covalently linked with Luminex^{®} color-coated beads.

To amplify the genes coding for immunoglobulin superfamily molecules, the inventor designed nested primers based on sequence information available in the public domain. For studying B and T cell VDJ rearrangement, the inventor designed primers to amplify rearranged and expressed RNAs. Generally, a pair of nested forward primers is designed from the V genes and a set of reverse nested primers are designed from the J or C genes. The average amplicon size is 250-350bp. For the IgHV genes, for example, there are 123 genes that can be classified into 7 different families, and the present primers are designed to be family-specific. However, if sequencing the amplified cDNA sequences, there are enough sequence diversities to allow further differentiation among the genes within the same family. For the MHC gene locus, the intent is to amplify genomic DNA.

The invention may be further described by means of the following non-limiting examples.

### Examples

### Amplification of T or B Cell Rearrangement Sites

All oligos were resuspended using 1x TE. All oligos except 454A and 454B were resuspended to a concentration of 100pmol/uL. 454A and 454B were resuspended to a concentration of 1000pmol/uL 454A and 454B are functionally the same as the common primers described previously, the different sequences were used for follow up high throughput sequencing procedures.

Three different primer mixes were made. An Alpha Delta primer mix included 82 primers (all of TRAV-C + TRDV-C), a Beta Gamma primer mix included 79 primers (all of TRBVC and TRGV-C) and a B cell primer mix that included a total of 70 primers. Fₒ, Fᵢ, and Rᵢ primers were at a concentration of 1pmol/µL. Rₒ primers were at a concentration of 5 pmol/uL. 454A and 454B were at a concentration of 30 pmol/µL.

Three different RNA samples were ordered from ALLCELLS (www.allcells.com). All samples were diluted down to a final concentration of 4 ng/uL. The sampes used were: ALL-PB-MNC (from a patient with acute lymphoblastic leukemia), NPB-Pan T Cells (normal T cells) and NPB-B Cells (normal B cells).

RT-PCR was performed using a Qiagen^{®} One-Step RT-PCR kit. Each sample contained the following:
10 µL of Qiagen^{®} Buffer
2 µL of DNTP's
2 µl of Enzyme
23.5 µL of dH2O
10 µL of the appropriate primer mix
2.5 µL of the appropriate template (10ng of RNA total)

The samples were run using the following cycling conditions:
50°C for 30 minutes
95°C for 15 minutes
94°C for 30 seconds
15 cycles of
55°C for 1 minute
72°C for 1 minute
94° C for 15 seconds
6 cycles of
70°C for 1 minute 30 seconds
94°C for 15 seconds
30 cycles of
55°C for 15 seconds
72°C for 15 seconds
72°C for 3 minutes
4°C Hold

The order of samples placed in the gel shown in Fig. 1a was: (1) Ladder (500bp being the largest working down in steps of 20bp, the middle bright band in Fig. 1a is 200bp); (2) α + δ primer mix with 10ng Pan T Cells Template; (3) β + γ primer mix with 10ng Pan T Cells Template; (4) B Cell primer mix with 10ng B Cells Template; (5) B Cell primer mix with 10ng ALL Cells Template; (6) α + δ primer mix with 10ng ALL Cells Template; (7) β + γ primer mix with 10ng ALL Cells Template; 8. α + δ primer mix blank; (9) β + γ primer mix blank; (10) B Cell primer mix blank; (11)Running buffer blank. These samples were run on a precast ClearPAGE^{®} SDS 10% gel using 1X ClearPAGE^{®} DNA native running buffer.

The initial experiment showed that a smear is generated from PCR reactions where templates were included. The smears indicate different sizes of PCR products were generated that represented a mixture of different VDJ rearrangements. There was some background amplification from the B cell reaction. Further improvement on that primer mix cleaned up the reaction.

To determine whether the PCR products indeed include different VDJ rearrangements, it was necessary to isolate and sequence the single clones. Instead of using the routine cloning procedures, the inventor used a different strategy. PCR products generated from the Alpha Delta mix and the Beta Gamma mix (lanes 2 and 3 in Fig. 1a) were diluted 1:1000 and a 2 µl aliquot used as PCR template in the following reaction. Then, instead of using a mixture of primers that targeting the entire repertoire, one pair of specific Fᵢ and Rᵢ primers were used (5 pmol each) to amplify only one specific PCR product. The following cycling conditions were used to amplify the samples:
95°C for 5 minutes
30 cycles of
94°C for 30 seconds
72°C for 1 minute
72 °C for 3 minutes
4°C hold

A Qiagen PCR kit was used to amplify the products. The Master Mix used for the PCR contained the following: 5µL 10x PCR Buffer, 1µL dNTP, 0.25µL HotStartTaq Plus, and 39.75 µL H₂O. (For a mix for 12 reactions: 60µL 10x PCR Buffer, 12µL dNTP, 3µL HotStartTaq Plus, and 477 µL H₂O.)

The photograph of the gel in Fig. 1b shows the PCR products of the following reactions: (1) Ladder; (2) TRAV1Fᵢ+TRACRᵢ with alpha delta Pan T PCR product; (3) TRAV2Fᵢ+TRACRᵢ with alpha delta Pan T PCR product; (4) TRAV3Fᵢ+TRACRᵢ with alpha delta Pan T PCR product; (5) TRAV4Fᵢ+TRACRᵢ with alpha delta Pan T PCR product; (6) TRAV5Fᵢ+TRACRᵢ with alpha delta Pan T PCR product; (7) TRAVlFᵢ+TRACRᵢ with alpha delta Pan T PCR product; (8) TRAV2Fᵢ+TRACRᵢ with alpha delta Pan T PCR product; (9) TRAV3Fᵢ+TRACRᵢ with alpha delta Pan T PCR product; (10) TRAV4Fᵢ+TRACRᵢ with alpha delta Pan T PCR product; (11) TRAV5Fᵢ+TRACRᵢ with alpha delta Pan T PCR product; (12) PCR Blank. Primers listed as F₁ are "forward inner" primers and primers listed as Fₒ are "forward outer" primers, with Ri and Rₒ indicating "reverse inner" and "reverse outer" primers, respectively.

As illustrated by Fig. 1b, a single PCR product was generated from each reaction. Different size bands were generated from different reactions. This PCR cloning approach is successful for two major reasons-(1) The PCR templates used in this reaction were diluted PCR products (1:1000) of previous reactions that used primer mixes to amplify all possible VDJ rearrangements (for example, a primer mix was used that included total of 82 primers to amplify T cell receptor Alpha and Delta genes) and (2) Only one pair of PCR primers, targeting a specific V gene, are used in each reaction during this "cloning" experiment. In every case, a single clone was obtained, and a specific T cell receptor V gene that matched the Fᵢ primer was identified.

### Sequencing of Immune Cell RNA Using Primers of SEQ ID NO: 1 - SEQ ID NO: 312

Pan-T, pan-B, and neutrophil isolation was performed using superparamagnetic polystyrene beads coated with monoclonal antibody specific for certain cell types (Dynabeads^{®}, Invitrogen Corp., Carlsbad, California) following manufacturer's instructions. Anti-CD3 beads were used to isolate pan-T cells, anti-CD19 beads for pan-B cells, and anti-CD15 beads for neutrophils. Isolated cells were resuspended in 300 µl RNAProtect^{®} (Qiagen) reagent and counted using a hemacytometer.

T cell subpopulations were isolated from a normal patient 48-year-old Asian male. PMBCs were obtained from 40 ml of whole blood collected in sodium heparin by density centrifugation over Ficoll Prep Plus Reagent. Pan-T cells were isolated from the mononuclear layer using a magnetic bead isolation kit (Miltenyi Biotec, Auburn, California), following manufacturer's instructions. Anti-CD4 and anti-CD25 beads were used to isolate regulatory T cells, anti-CD56 for NKT cells, anti-CD8 for cytotoxic T cells, and anti-CD4 and anti-CD294 for Th2 cells. Th1 cells were isolated via negative selection. A separate 40 ml sample of whole blood collected in sodium heparin was used to obtain naive, activated, and memory T cell subpopulations. Anti-CD45RA beads were used to isolate naive T cells, anti-CD69 for activated T cells and anti-CD45RO beads for memory T cells. Isolated cells were re-suspended in 300 µl of RNAProtect^{®} reagent (Qiagen). Cells were counted using a hemacytometer.

DNA extraction from the isolated neutrophils was performed using a QIAmp^{®} DNA mini kit (Qiagen) using the protocol provided by the manufacturer. RNA was extracted from T cell subsets using an RNeasy^{®} kit (Qiagen) according to the protocol provided by the manufacturer. The concentrations of extracted DNA and RNA were measured using Nanodrop^{®} technology (Nanodrop Technologies, Wilmington, Delaware). Samples were stored at -80°C.

RT-PCR was performed according to the method of the invention using nested PCR to amplify multiple targets and target-specific primers to incorporate a common primer binding sequence into the resulting amplicons in a first amplification reaction. Common primers were then used in a second amplification reaction to exponentially amplify the amplicons rescued from the first amplification reaction while preserving the relative ratios of each amplicon. PCR was performed using a One-Step RT-PCR kit (Qiagen). DNA amplification for HLA typing was similarly performed, but with a mulitplex PCR kit (Qiagen). Each amplicon mixture was subjected to high-throughput sequencing with the Roche 454 sequencing platform.

More than 1.6 million effective sequences were generated for one single individual (normal 48-year-old Asian male) by sampling different subpopulations of lymphocytes in peripheral blood at different time points. Additionally, 170,734 effective sequences were generated for the colon cancer, CLL, SLE, and a second healthy patient (a 32-year-old Caucasian male). The number of unique reads generated in this study was compared to the number of unique reads existing in public databases in Table 1. The public sequence data set was compiled by searching Genbank nucleotide database with terms of 'human[orgn] AND (immunoglobulin[titl] OR T-cell receptor[titl]) AND mRNA[titl]'. In addition, the annotated IMGT/LIGM-DB (Brezinschek *et al*, 1995) cDNA sequences were gathered with a Python script. The two data sets were merged, and one copy was kept for any redundant sequences.

Biased usage of V, and J gene segments in a healthy control, CLL, colon cancer, and SLE sample was analyzed. The bias of domain usage was particularly outstanding for TCR beta chain in the colon cancer sample and SLE sample, while in the healthy control sample the domain usage is quite normal without significant bias to any particular domain. It was evident that colon cancer and SLE profiles not only show clonal expansion, but demonstrate the loss of overall diversity, as well.

The distribution of functional germline V, J gene segments seen in the pan-T and pan-B populations from normal patient indicated that 87.2% of potential combinations have sequences observed. Only IGHV3-d was not observed in this investigation, while TRBV4-3, IGHV3-d, IGHV4-30-4 and IGHV4-31 and IGHL3-22 were observed in other samples with extremely low frequency. Previous research did not reveal any cDNA sequence data related to IGHV3-d, which suggests that IGHV3-d may be used infrequently. Some sequences were present in high (e.g. 1000) numbers, while others were present in significantly lower numbers. The inventor believes that higher numbers represent lymphocyte clonal expansions, reflecting the real immune responses in the subject. Studies of VH gene distribution in normal individuals have previously found the frequency of usage in general to be similar to the germline complexity, while many immune responses show some level of bias in the usage of V, D and J gene segments.

**Table 1**

| Primer | Sequence | Sequence ID Number |
|---|---|---|
| | | |
| TRAV1Fo | 5'-TGCACGTACCAGACATCTGG-3' | SEQ ID NO: 1 |
| TRAV1Fi | AGGTCGTTTTTCTTCATTCC | SEQ ID NO: 2 |
| TRAV2Fo | TCTGTAATCACTCTGTGTCC | SEQ ID NO: 3 |
| TRAV2Fi | AGGGACGATACAACATGACC | SEQ ID NO: 4 |
| TRAV3Fo | CTATTCAGTCTCTGGAAACC | SEQ ID NO: 5 |
| TRAV3Fi | ATACATCACAGGGGATAACC | SEQ ID NO: 6 |
| TRAV4Fo | TGTAGCCACAACAACATTGC | SEQ ID NO: 7 |
| TRAV4Fi | AAAGTTACAAACGAAGTGGC | SEQ ID NO: 8 |
| TRAV5Fo | GCACTTACACAGACAGCTCC | SEQ ID NO: 9 |
| TRAV5Fi | TATGGACATGAAACAAGACC | SEQ ID NO: 10 |
| TRAV6Fo | GCAACTATACAAACTATTCC | SEQ ID NO: 11 |
| TRAV6Fi | GTTTTCTTGCTACTCATACG | SEQ ID NO: 12 |
| TRAV7Fo | TGCACGTACTCTGTCAGTCG | SEQ ID NO: 13 |
| TRAV7Fi | GGATATGAGAAGCAGAAAGG | SEQ ID NO: 14 |
| TRAV8Fo | AATCTCTTCTGGTATGTSCA | SEQ ID NO: 15 |
| TRAV8Fi | GGYTTTGAGGCTGAATTTA | SEQ ID NO: 16 |
| TRAV9Fo | GTCCAATATCCTGGAGAAGG | SEQ ID NO: 17 |
| TR.AV9Fi | AACCACTTCTTTCCACTTGG | SEQ ID NO: 18 |
| TRAV10Fo | AATGCAATTATACAGTGAGC | SEQ ID NO: 19 |
| TRAV10Fi | TGAGAACACAAAGTCGAACG | SEQ ID NO: 20 |
| TRAV11Fo | TCTTAATTGTACTTATCAGG | SEQ ID NO: 21 |
| TRAV11Fi | TCAATCAAGCCAGAAGGAGC | SEQ ID NO: 22 |
| TRAV12Fo | TCAGTGTTCCAGAGGGAGCC | SEQ ID NO: 23 |
| TRAV12Fi | ATGGAAGGTTTACAGCACAG | SEQ ID NO: 24 |
| TRAV13Fo | ACCCTGAGTGTCCAGGAGGG | SEQ ID NO: 25 |
| TRAV13Fi | TTATAGACATTCGTTCAAAT | SEQ ID NO: 26 |
| TRAV14Fo | TGGACTGCACATATGACACC | SEQ ID NO: 27 |
| TRAV14Fi | CAGCAAAATGCAACAGAAGG | SEQ ID NO: 28 |
| TRAV16Fo | AGCTGAAGTGCAACTATTCC | SEQ ID NO: 29 |
| TRAV16Fi | TCTAGAGAGAGCATCAAAGG | SEQ ID NO: 30 |
| TRAV17Fo | AATGCCACCATGAACTGCAG | SEQ ID NO: 31 |
| TRAV17Fi | GAAAGAGAGAAACACAGTGG | SEQ ID NO: 32 |
| TRAV18Fo | GCTCTGACATTAAACTGCAC | SEQ ID NO: 33 |
| TRAV18Fi | CAGGAGACGGACAGCAGAGG | SEQ ID NO: 34 |
| TRAV19Fo | ATGTGACCTTGGACTGTGTG | SEQ ID NO: 35 |
| TRAV19Fi | GAGCAAAATGAAATAAGTGG | SEQ ID NO: 36 |
| TRAV20Fo | ACTGCAGTTACACAGTCAGC | SEQ ID NO: 37 |
| TRAV20Fi | AGAAAGAAAGGCTAAAAGCC | SEQ ID NO: 38 |
| TRAV21Fo | ACTGCAGTTTCACTGATAGC | SEQ ID NO: 39 |
| TRAV21Fi | CAAGTGGAAGACTTAATGCC | SEQ ID NO: 40 |
| TRAV22Fo | GGGAGCCAATTCCACGCTGC | SEQ ID NO: 41 |
| TRAV22Fi | ATGGAAGATTAAGCGCCACG | SEQ ID NO: 42 |
| TRAV23Fo | ATTTCAATTATAAACTGTGC | SEQ ID NO: 43 |
| TRAV23Fi | AAGGAAGATTCACAATCTCC | SEQ ID NO: 44 |
| TRAV24Fo | GCACCAATTTCACCTGCAGC | SEQ ID NO: 45 |
| TRAV24Fi | AGGACGAATAAGTGCCACTC | SEQ ID NO: 46 |
| TRAV25Fo | TCACCACGTACTGCAATTCC | SEQ ID NO: 47 |
| TRAV25Fi | AGACTGACATTTCAGTTTGG | SEQ ID NO: 48 |
| TRAV26Fo | TCGACAGATTCMCTCCCAGG | SEQ ID NO: 49 |
| TRAV26Fi | GTCCAGYACCTTGATCCTGC | SEQ ID NO: 50 |
| TRAV27Fo | CCTCAAGTGTTTTTTCCAGC | SEQ ID NO: 51 |
| TRAV27Fi | GTGACAGTAGTTACGGGTGG | SEQ ID NO: 52 |
| TRAV29Fo | CAGCATGTTTGATTATTTCC | SEQ ID NO: 53 |
| TRAV29Fi | ATCTATAAGTTCCATTAAGG | SEQ ID NO: 54 |
| TRAV30Fo | CTCCAAGGCTTTATATTCTG | SEQ ID NO: 55 |
| TRAV30Fi | ATGATATTACTGAAGGGTGG | SEQ ID NO: 56 |
| TRAV34Fo | ACTGCACGTCATCAAAGACG | SEQ ID NO: 57 |
| TRAV34Fi | TTGATGATGCTACAGAAAGG | SEQ ID NO: 58 |
| TRAV35Fo | TGAACTGCACTTCTTCAAGC | SEQ ID NO: 59 |
| TRAV35Fi | CTTGATAGCCTTATATAAGG | SEQ ID NO: 60 |
| TRAV36Fo | TCAATTGCAGTTATGAAGTG | SEQ ID NO: 61 |
| TRAV36Fi | TTTATGCTAACTTCAAGTGG | SEQ ID NO: 62 |
| TRAV38Fo | GCACATATGACACCAGTGAG | SEQ ID NO: 63 |
| TRAV38Fi | TCGCCAAGAAGCTTATAAGC | SEQ ID NO: 64 |
| TRAV39Fo | TCTACTGCAATTATTCAACC | SEQ ID NO: 65 |
| TRAV39Fi | CAGGAGGGACGATTAATGGC | SEQ ID NO: 66 |
| TRAV40Fo | TGAACTGCACATACACATCC | SEQ ID NO: 67 |
| TRAV40Fi | ACAGCAAAAACTTCGGAGGC | SEQ ID NO: 68 |
| TRAV41Fo | AACTGCAGTTACTCGGTAGG | SEQ ID NO: 69 |
| TRAV41Fi | AAGCATGGAAGATTAATTGC | SEQ ID NO: 70 |
| | | |
| TRACRo | GCAGACAGACTTGTCACTGG | SEQ ID NO: 71 |
| TRACRi | AGTCTCTCAGCTGGTACACG | SEQ ID NO: 72 |
| TRBV1Fo | AATGAAACGTGAGCATCTGG | SEQ ID NO: 73 |
| TRBV1Fi | CATTGAAAACAAGACTGTGC | SEQ ID NO: 74 |
| TRBV2Fo | GTGTCCCCATCTCTAATCAC | SEQ ID NO: 75 |
| TRBV2Fi | TGAAATCTCAGAGAAGTCTG | SEQ ID NO: 76 |
| TRBV3Fo | TATGTATTGGTATAAACAGG | SEQ ID NO: 77 |
| TRBV3Fi | CTCTAAGAAATTTCTGAAGA | SEQ ID NO: 78 |
| TRBV4Fo | GTCTTTGAAATGTGAACAAC | SEQ ID NO: 79 |
| TRBV4Fi | GGAGCTCATGTTTGTCTACA | SEQ ID NO: 80 |
| TRBV5Fo | GATCAAAACGAGAGGACAGC | SEQ ID NO: 81 |
| TRBV5aFi | CAGGGGCCCCAGTTTATCTT | SEQ ID NO: 82 |
| TRBV5bFi | GAAACARAGGAAACTTCCCT | SEQ ID NO: 83 |
| TRBV6aFo | GTGTGCCCAGGATATGAACC | SEQ ID NO: 84 |
| TRBV6bFo | CAGGATATGAGACATAATGC | SEQ ID NO: 85 |
| TRBV6aFi | GGTATCGACAAGACCCAGGC | SEQ ID NO: 86 |
| TRBV6bFi | TAGACAAGATCTAGGACTGG | SEQ ID NO: 87 |
| TRBV7Fo | CTCAGGTGTGATCCAATTTC | SEQ ID NO: 88 |
| TRBV7aFi | TCTAATTTACTTCCAAGGCA | SEQ ID NO: 89 |
| TRBV7bFi | TCCCAGAGTGATGCTCAACG | SEQ ID NO: 90 |
| TRBV7cFi | ACTTACTTCAATTATGAAGC | SEQ ID NO: 91 |
| TRBV7dFi | CCAGAATGAAGCTCAACTAG | SEQ ID NO: 92 |
| TRBV9Fo | GAGACCTCTCTGTGTACTGG | SEQ ID NO: 93 |
| TRBV9Fi | CTCATTCAGTATTATAATGG | SEQ ID NO: 94 |
| TRBV10Fo | GGAATCACCCAGAGCCCAAG | SEQ ID NO: 95 |
| TRBV10Fi | GACATGGGCTGAGGCTGATC | SEQ ID NO: 96 |
| TRBV11Fo | CCTAAGGATCGATTTTCTGC | SEQ ID NO: 97 |
| TRBV11Fi | ACTCTCAAGATCCAGCCTGC | SEQ ID NO: 98 |
| TRBV12Fo | AGGTGACAGAGATGGGACAA | SEQ ID NO: 99 |
| TRBV12aFi | TGCAGGGACTGGAATTGCTG | SEQ ID NO: 100 |
| TRBV12bFi | GTACAGACAGACCATGATGC | SEQ ID NO: 101 |
| TRBV13Fo | CTATCCTATCCCTAGACACG | SEQ ID NO: 102 |
| TRBV13Fi | AAGATGCAGAGCGATAAAGG | SEQ ID NO: 103 |
| TRBV14Fo | AGATGTGACCCAATTTCTGG | SEQ ID NO: 104 |
| TRBV14Fi | AGTCTAAACAGGATGAGTCC | SEQ ID NO: 105 |
| TRBV15Fo | TCAGACTTTGAACCATAACG | SEQ ID NO: 106 |
| TRBV15Fi | AAAGATTTTAACAATGAAGC | SEQ ID NO: 107 |
| TRBV16Fo | TATTGTGCCCCAATAAAAGG | SEQ ID NO: 108 |
| TRBV16Fi | AATGTCTTTGATGAAACAGG | SEQ ID NO: 109 |
| TRBV17Fo | ATCCATCTTCTGGTCACATG | SEQ ID NO: 110 |
| TRBV17Fi | AACATTGCAGTTGATTCAGG | SEQ ID NO: 111 |
| TRBV18Fo | GCAGCCCAATGAAAGGACAC | SEQ ID NO: 112 |
| TRBV18Fi | AATATCATAGATGAGTCAGG | SEQ ID NO: 113 |
| TRBV19Fo | TGAACAGAATTTGAACCACG | SEQ ID NO: 114 |
| TRBV19Fi | TTTCAGAAAGGAGATATAGC | SEQ ID NO: 115 |
| TRBV20Fo | TCGAGTGCCGTTCCCTGGAC | SEQ ID NO: 116 |
| TRBV20Fi | GATGGCAACTTCCAATGAGG | SEQ ID NO: 117 |
| TRBV21Fo | GCAAAGATGGATTGTGTTCC | SEQ ID NO: 118 |
| TRBV21Fi | CGCTGGAAGAAGAGCTCAAG | SEQ ID NO: 119 |
| TRBV23Fo | CATTTGGTCAAAGGAAAAGG | SEQ ID NO: 120 |
| TRBV23Fi | GAATGAACAAGTTCTTCAAG | SEQ ID NO: 121 |
| TRBV24Fo | ATGCTGGAATGTTCTCAGAC | SEQ ID NO: 122 |
| TRBV24Fi | GTCAAAGATATAAACAAAGG | SEQ ID NO: 123 |
| TRBV25Fo | CTCTGGAATGTTCTCAAACC | SEQ ID NO: 124 |
| TRBV25Fi | TAATTCCACAGAGAAGGGAG | SEQ ID NO: 125 |
| TRBV26Fo | CCCAGAATATGAATCATGTT | SEQ ID NO: 126 |
| TRBV26Fi | ATTCACCTGGCACTGGGAGC | SEQ ID NO: 127 |
| TRBV27Fo | TTGTTCTCAGAATATGAACC | SEQ ID NO: 128 |
| TRBV27Fi | TGAGGTGACTGATAAGGGAG | SEQ ID NO: 129 |
| TRBV28Fo | ATGTGTCCAGGATATGGACC | SEQ ID NO: 130 |
| TRBV28Fi | AAAAGGAGATATTCCTGAGG | SEQ ID NO: 131 |
| TRBV29Fo | TCACCATGATGTTCTGGTAC | SEQ ID NO: 132 |
| TRBV29Fi | CTGGACAGAGCCTGACACTG | SEQ ID NO: 133 |
| TRBV30Fo | TGTGGAGGGAACATCAAACC | SEQ ID NO: 134 |
| TRBV30Fi | TTCTACTCCGTTGGTATTGG | SEQ ID NO: 135 |
| | | |
| TRBCRo | GTGTGGCCTTTTGGGTGTGG | SEQ ID NO: 136 |
| TRBCRi | TCTGATGGCTCAAACACAGC | SEQ ID NO: 137 |
| | | |
| TRDV1Fo | TGTATGAAACAAGTTGGTGG | SEQ ID NO: 138 |
| TRDV1Fi | CAGAATGCAAAAAGTGGTCG | SEQ ID NO: 139 |
| TRDV2Fo | ATGAAAGGAGAAGCGATCGG | SEQ ID NO: 140 |
| TRDV2Fi | TGGTTTCAAAGACAATTTCC | SEQ ID NO: 141 |
| TRDV3Fo | GACACTGTATATTCAAATCC | SEQ ID NO: 142 |
| TRDV3Fi | GCAGATTTTACTCAAGGACG | SEQ ID NO: 143 |
| | | |
| TRDCRo | AGACAAGCGACATTTGTTCC | SEQ ID NO: 144 |
| TRDCRi | ACGGATGGTTTGGTATGAGG | SEQ ID NO: 145 |
| | | |
| TRGV1-5Fo | GGGTCATCTGCTGAAATCAC | SEQ ID NO: 146 |
| TRGV1-5,8Fi | AGGAGGGGAAGGCCCCACAG | SEQ ID NO: 147 |
| TRGV8Fo | GGGTCATCAGCTGTAATCAC | SEQ ID NO: 148 |
| TRGV5pFi | AGGAGGGGAAGACCCCACAG | SEQ ID NO: 149 |
| TRGV9Fo | AGCCCGCCTGGAATGTGTGG | SEQ ID NO: 150 |
| TRGV9Fi | GCACTGTCAGAAAGGAATCC | SEQ ID NO: 151 |
| TRGV10Fo | AAGAAAAGTATTGACATACC | SEQ ID NO: 152 |
| TRGV10Fi | ATATTGTCTCAACAAAATCC | SEQ ID NO: 153 |
| TRGV11Fo | AGAGTGCCCACATATCTTGG | SEQ ID NO: 154 |
| TRGV11Fi | GCTCAAGATTGCTCAGGTGG | SEQ ID NO: 155 |
| | | |
| TRGCRo | GGATCCCAGAATCGTGTTGC | SEQ ID NO: 156 |
| TRGCRi | GGTATGTTCCAGCCTTCTGG | SEQ ID NO: 157 |

**Table 2**

| **Primer** | **Sequence** | **Sequence ID Number** |
|---|---|---|
| IgHV1aFo | AGTGAAGGTCTCCTGCAAGG | SEQ ID NO: 158 |
| IgHV1bFo | AGTGAAGGTTTCCTGCAAGG | SEQ ID NO: 159 |
| IgHV1aFi | AGTTCCAGGGCAGAGTCAC | SEQ ID NO: 160 |
| IgHV1bFi | AGTTTCAGGGCAGGGTCAC | SEQ ID NO: 161 |
| IgHV1cFi | AGTTCCAGGAAAGAGTCAC | SEQ ID NO: 162 |
| IgHV1dFi | AATTCCAGGACAGAGTCAC | SEQ ID NO: 163 |
| IgHV2Fo | TCTCTGGGTTCTCACTCAGC | SEQ ID NO: 164 |
| IgHV2Fi | AAGGCCCTGGAGTGGCTTGC | SEQ ID NO: 165 |
| IgHV3aFo | TCCCTGAGACTCTCCTGTGC | SEQ ID NO: 166 |
| IgHV3bFo | CTCTCCTGTGCAGCCTCTGG | SEQ ID NO: 167 |
| IgHV3cFo | GGTCCCTGAGACTCTCCTGT | SEQ ID NO: 168 |
| IgHV3dFo | CTGAGACTCTCCTGTGTAGC | SEQ ID NO: 169 |
| IgHV3aFi | CTCCAGGGAAGGGGCTGG | SEQ ID NO: 170 |
| IgHV3bFi | GGCTCCAGGCAAGGGGCT | SEQ ID NO: 171 |
| IgHV3cFi | ACTGGGTCCGCCAGGCTCC | SEQ ID NO: 172 |
| IgHV3dFi | GAAGGGGCTGGAGTGGGT | SEQ ID NO: 173 |
| IgHV3eFi | AAAAGGTCTGGAGTGGGT | SEQ ID NO: 174 |
| IgHV4Fo | AGACCCTGTCCCTCACCTGC | SEQ ID NO: 175 |
| IgHV4Fi | AGGGVCTGGAGTGGATTGGG | SEQ ID NO: 176 |
| IgHV5Fo | GCGCCAGATGCCCGGGAAAG | SEQ ID NO: 177 |
| IgHV5Fi | GGCCASGTCACCATCTCAGC | SEQ ID NO: 178 |
| IgHV6Fo | CCGGGGACAGTGTCTCTAGC | SEQ ID NO: 179 |
| IgHV6Fi | GCCTTGAGTGGCTGGGAAGG | SEQ ID NO: 180 |
| IgHV7Fo | GTTTCCTGCAAGGCTTCTGG | SEQ ID NO: 181 |
| IgHV7Fi | GGCTTGAGTGGATGGGATGG | SEQ ID NO: 182 |
| | | |
| IgHJRo | ACCTGAGGAGACGGTGACC | SEQ ID NO: 183 |
| IgHJ1Ri | CAGTGCTGGAAGTATTCAGC | SEQ ID NO: 184 |
| IgHJ2Ri | AGAGATCGAAGTACCAGTAG | SEQ ID NO: 185 |
| IgHJ3Ri | CCCCAGATATCAAAAGCATC | SEQ ID NO: 186 |
| IgHJ4Ri | GGCCCCAGTAGTCAAAGTAG | SEQ ID NO: 187 |
| IgHJ5Ri | CCCAGGGGTCGAACCAGTTG | SEQ ID NO: 188 |
| IgHJ6Ri | CCCAGACGTCCATGTAGTAG | SEQ ID NO: 189 |
| | | |
| IgKV1Fo | TAGGAGACAGAGTCACCATC | SEQ ID NO: 190 |
| IgKV1Fi | TTCAGYGRCAGTGGATCTGG | SEQ ID NO: 191 |
| IgKV2Fo | GGAGAGCCGGCCTCCATCTC | SEQ ID NO: 192 |
| IgKV2aFi | TGGTACCTGCAGAAGCCAGG | SEQ ID NO: 193 |
| IgKV2bFi | CTTCAGCAGAGGCCAGGCCA | SEQ ID NO: 194 |
| IgKV3-7Fo | GCCTGGTACCAGCAGAAACC | SEQ ID NO: 195 |
| IgKV3Fi | GCCAGGTTCAGTGGCAGTGG | SEQ ID NO: 196 |
| IgKV6-7Fi | TCGAGGTTCAGTGGCAGTGG | SEQ ID NO: 197 |
| IgKV4-5Fi | GACCGATTCAGTGGCAGCGG | SEQ ID NO: 198 |
| | | |
| IgKCRo | TTCAACTGCTCATCAGATGG | SEQ ID NO: 199 |
| IgKCRi | ATGAAGACAGATGGTGCAGC | SEQ ID NO: 200 |
| | | |
| IgLV1aFo | GGGCAGAGGGTCACCATCTC | SEQ ID NO: 201 |
| IgLV1bFo | GGACAGAAGGTCACCATCTC | SEQ ID NO: 202 |
| IgLV1aFi | TGGTACCAGCAGCTCCCAGG | SEQ ID NO: 203 |
| IgLV1bFi | TGGTACCAGCAGCTTCCAGG | SEQ ID NO: 204 |
| IgLV2Fo | CTGCACTGGAACCAGCAGTG | SEQ ID NO: 205 |
| IgLV2Fi | TCTCTGGCTCCAAGTCTGGC | SEQ ID NO: 206 |
| IgLV3aFo | ACCAGCAGAAGCCAGGCCAG | SEQ ID NO: 207 |
| IgLV3bFo | GAAGCCAGGACAGGCCCCTG | SEQ ID NO: 208 |
| IgLV3aFi | CTGAGCGATTCTCTGGCTCC | SEQ ID NO: 209 |
| IgLV3bFi | TTCTCTGGGTCCACCTCAGG | SEQ ID NO: 210 |
| IgLV3cFi | TTCTCTGGCTCCAGCTCAGG | SEQ ID NO: 211 |
| IgLV4Fo | TCGGTCAAGCTCACCTGCAC | SEQ ID NO: 212 |
| IgLV4Fi | GGGCTGACCGCTACCTCACC | SEQ ID NO: 213 |
| IgLV5Fo | CAGCCTGTGCTGACTCAGCC | SEQ ID NO: 214 |
| IgLV5Fi | CCAGCCGCTTCTCTGGATCC | SEQ ID NO: 215 |
| IgLV6Fo | CCATCTCCTGCACCCGCAGC | SEQ ID NO: 216 |
| IgLV7-8Fo | TCCCCWGGAGGGACAGTCAC | SEQ ID NO: 217 |
| IgLV9, 11Fo | CTCMCCTGCACCCTGAGCAG | SEQ ID NO: 218 |
| IgLV10Fo | AGACCGCCACACTCACCTGC | SEQ ID NO: 219 |
| IgLV6,8Fi | CTGATCGSTTCTCTGGCTCC | SEQ ID NO: 220 |
| IgLV7Fi | CTGCCCGGTTCTCAGGCTCC | SEQ ID NO: 221 |
| IgLV9Fi | ATCCAGGAAGAGGATGAGAG | SEQ ID NO: 222 |
| IgLV10-11Fi | CTCCAGCCTGAGGACGAGGC | SEQ ID NO: 223 |
| IgLC1-7Ro | GCTCCCGGGTAGAAGTCACT | SEQ ID NO: 224 |
| IgLC1-7Ri | AGTGTGGCCTTGTTGGCTTG | SEQ ID NO: 225 |

**Table 3**

| **Primer** | **Sequence** | **Sequence ID Number** |
|---|---|---|
| HLAI Fo11 | CCCACTCCATGAGGTATTTC | SEQ ID NO: 226 |
| HLAI Fo12 | CCTACTCCATGAGGTATTTC | SEQ ID NO: 227 |
| HLAI Fo31 | GCGGGGAGCCCCGCTTCATC | SEQ ID NO: 228 |
| HLAI Fo32 | GCGGGAAGCCCCGCTTCATC | SEQ ID NO: 229 |
| HLAI Fo33 | GTGGAGAGCCCCGCTTCATC | SEQ ID NO: 230 |
| HLAI Fo34 | GCGGAAAGCCCCGCTTCATC | SEQ ID NO: 231 |
| HLAI Fo35 | GCGGAAAGCCCCACTTCATC | SEQ ID NO: 232 |
| HLAI Fo36 | GCGGGAAGCCCCACTTCATC | SEQ ID NO: 233 |
| HLAI Fi11 | GTGGGCTACGTGGACGACAC | SEQ ID NO: 234 |
| HLAI Fi12 | GTGGGCTACGTGGACGGCAC | SEQ ID NO: 235 |
| HLAI-Fi21 | GTTCGTGCGGTTCGACAGCG | SEQ ID NO: 236 |
| HLAI-Fi22 | GTTCGTGCGGTTTGACAGCG | SEQ ID NO: 237 |
| HLAI-Fi23 | GTTCGTGAGGTTCGACAGCG | SEQ ID NO: 238 |
| HLAI Ri11 | TAATCCTTGCCGTCGTAGGC | SEQ ID NO: 239 |
| HLAI Ri12 | TAATCCTTGCCGTCGTAAGC | SEQ ID NO: 240 |
| HLAI Ri13 | TAATCTTTGCCGTCGTAGGC | SEQ ID NO: 241 |
| | | |
| HLAI Ro11 | GGTCCTCGTTCAGGGCGATG | SEQ ID NO: 242 |
| HLAI Ro12 | GGTCCTCTTTCAGGGCGATG | SEQ ID NO: 243 |
| HLAI Ro13 | GGTCCTCGTTCAAGGCGATG | SEQ ID NO: 244 |
| HLAI Ro14 | GATCCTCGTTCAGGGCGATG | SEQ ID NO: 245 |
| HLAI Ro15 | GGTCCTCATTCAGGGCGATG | SEQ ID NO: 246 |
| | | |
| HLAI-Fo41 | GCCTACGACGGCAAGGATTA | SEQ ID NO: 247 |
| HLAI-Fo42 | GCTTACGACGGCAAGGATTA | SEQ ID NO: 248 |
| HLAI-Fo43 | GCCTACGACGGCAAAGATTA | SEQ ID NO: 249 |
| HLAI-Fi31 | CATCGCCCTGAACGAGGACC | SEQ ID NO: 250 |
| HLAI-Fi32 | CATCGCCCTGAAAGAGGACC | SEQ ID NO: 251 |
| HLAI-Fi33 | CATCGCCTTGAACGAGGACC | SEQ ID NO: 252 |
| HLAI-Fi34 | CATCGCCCTGAACGAGGATC | SEQ ID NO: 253 |
| HLAI-Fi35 | CATCGCCCTGAATGAGGACC | SEQ ID NO: 254 |
| HLAI-Ri21 | GGTATCTGCGGAGCCCGTCC | SEQ ID NO: 255 |
| HLAI-Ri22 | GGTATCTGCGGAGCCACTCC | SEQ ID NO: 256 |
| HLAI-Ri23 | GGTGTCTGCGGAGCCACTCC | SEQ ID NO: 257 |
| HLAI-Ri24 | GGTATCCGCGGAGCCACTCC | SEQ ID NO: 258 |
| HLAI-Ro21 | GCAGCGTCTCCTTCCCGTTC | SEQ ID NO: 259 |
| HLAI-Ro22 | CCAGCTTGTCCTTCCCGTTC | SEQ ID NO: 260 |
| HLAI-Ro23 | CCAGCGTGTCCTTCCCGTTC | SEQ ID NO: 261 |
| HLAI-Ro24 | GCAGCGTCTCCTTCCCATTC | SEQ ID NO: 262 |
| HLAI-Ro25 | GCAGCGTCTCCTTCCKGTTC | SEQ ID NO: 263 |
| | | |
| DRB17 Fo11 | TGTCATTTCTTCAATGGGAC | SEQ ID NO: 264 |
| DRB1 Fo12 | AGTGTCATTTCTTCAACGGG | SEQ ID NO: 265 |
| DRB1 Fo13 | GTGTTATTTCTTCAATGGGA | SEQ ID NO: 266 |
| DRB1 Fo14 | GTGTCAATTCTTCAATGGGA | SEQ ID NO: 267 |
| DRB4 Fo | GTGTCATTTCCTCAATGGGA | SEQ ID NO: 268 |
| DRB1 Fi11 | GGAGCGGGTGCGGTTGCTGG | SEQ ID NO: 269 |
| DRB1 Fi12 | GGAGCGGGTGCGGTACCTGG | SEQ ID NO: 270 |
| DRB1 Fi13 | GGAGCGGGTGCGGTTCCTGG | SEQ ID NO: 271 |
| DRB1 Fi14 | GGAGCGGGTGCGATTCCTGG | SEQ ID NO: 272 |
| DRB1 Fi15 | GGAGCGGGTGCGGTATCTGC | SEQ ID NO: 273 |
| DRB1 Fi16 | GGAGCGGGTGCGGTTACTGG | SEQ ID NO: 274 |
| DRB45 Fi | ACATCTATAACCAAGAGGAG | SEQ ID NO: 275 |
| DRB6 Fi | ACATCCATAAACGGGAGGAG | SEQ ID NO: 276 |
| DRB7 Fi | TATAACCAAGAGGAGTACGT | SEQ ID NO: 277 |
| | | |
| DRB Ri11 | AACCCCGTAGTTGTGTCTGC | SEQ ID NO: 278 |
| DRB4 Ri | AACCCCGTAGTTGTATCTGC | SEQ ID NO: 279 |
| DRB6 Ri | CGTAATTGTATCTGCAGTAG | SEQ ID NO: 280 |
| DRB7 Ri | TAGTTGTCCACTTCGGCCCG | SEQ ID NO: 281 |
| DRB Ro1 | CGCTGCACTGTGAAGCTCTC | SEQ ID NO: 282 |
| DRB Ro2 | CGCTGCACCGTGAAGCTCTC | SEQ ID NO: 283 |
| DRA Fo | CCTGTGGAACTGAGAGAGCC | SEQ ID NO: 284 |
| DRA Fi | CAACGTCCTCATCTGTTTCA | SEQ ID NO: 285 |
| DRA Ri | CTGCTGCATTGCTTTTGCGC | SEQ ID NO: 286 |
| DRA Ro | TTACAGAGGCCCCCTGCGTT | SEQ ID NO: 287 |
| DPB Fo | GTCCAGGGCAGGGCCACTCC | SEQ ID NO: 288 |
| DPB Fi11 | AATTACGTGTACCAGGGACG | SEQ ID NO: 289 |
| DPB Fi12 | AATTACCTTTTCCAGGGACG | SEQ ID NO: 290 |
| DPB Fi13 | AATTACGTGTACCAGTTACG | SEQ ID NO: 291 |
| DPB Fi14 | AATTACGCGTACCAGTTACG | SEQ ID NO: 292 |
| DPB Ri11 | CGGCCTCGTCCAGCTCGTAG | SEQ ID NO: 293 |
| DPB Ri12 | TGGGCCCGCCCAGCTCGTAG | SEQ ID NO: 294 |
| DPB Ri13 | TGGGCCCGACCAGCTCGTAG | SEQ ID NO: 295 |
| DPB Ro | GGACTCGGCGCTGCAGGTC | SEQ ID NO: 296 |
| DPA Fo | AGGAGCTGGGGCCATCAAGG | SEQ ID NO: 297 |
| DPA Fi | GACCATGTGTCAACTTATGC | SEQ ID NO: 298 |
| DPA Ri1 | CTCAGGGGGATCGTTGGTGG | SEQ ID NO: 299 |
| DPA Ri2 | CTCAGGGGGATCATTGGCGG | SEQ ID NO: 300 |
| DPA Ro | CAGCTCCACAGGCTCCTTGG | SEQ ID NO: 301 |
| | | |
| DQB Fo | ACTCTCCCGAGGATTTCGTG | SEQ ID NO: 302 |
| DQB Fi | TGTGCTACTTCACCAACGGG | SEQ ID NO: 303 |
| DQB Ri1 | ACCTCGTAGTTGTGTCTGCA | SEQ ID NO: 304 |
| DQB Ri2 | AACTGGTAGTTGTGTCTGCA | SEQ ID NO: 305 |
| DQB Ro1 | ACTCTCCTCTGCAGGATCCC | SEQ ID NO: 306 |
| DQB Ro2 | ACTCGCCGCTGCAAGGTCGT | SEQ ID NO: 307 |
| DQB Ro3 | ACTCTCCTCTGCAAGATCCC | SEQ ID NO: 308 |
| DQA Fo | TGGTCTAAACTTGTACCAGT | SEQ ID NO: 309 |
| DQA Fi | ACCATGAATTTGATGGAGA | SEQ ID NO: 310 |
| DQA Ri | GGAACCTCATTGGTAGCAGC | SEQ ID NO: 311 |
| DQA Ro | ACTTGGAAAACACTGTGACC | SEQ ID NO: 312 |

### Organization Applicant

Street :
City :
State :
Country :
PostalCode :
PhoneNumber :
FaxNumber :
EmailAddress :
   <110> OrganizationName : Hudson Alpha Institute for Biotechnology

### Individual Applicant

Street :
City :
State :
Country :
PostalCode :
PhoneNumber :
FaxNumber :
EmailAddress :
   <110> LastName : Han
      <110> FirstName : Jian
      <110> MiddleInitial :
      <110> Suffix :

### Application Project

<120> Title : METHOD FOR EVALUATING AND COMPARING IMMUNOREPERTOIRES
<130> AppFileReference : HA-IR
<140> CurrentAppNumber :
<141> CurrentFilingDate : ___-_-_

### Sequence

<213> OrganismName : Artificial Sequence
<400> PreSequenceString :
   tgcacgtacc agacatctgg 20
<212> Type : DNA
<211> Length : 20
   SequenceName : SEQ ID NO: 1
   SequenceDescription :

### Feature

Sequence: SEQ ID NO: 1:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aggtcgtttt tcttcattcc 20
<212> Type : DNA
<211> Length : 20
   SequenceName : SEQ ID NO: 2

### SequenceDescription :

### Feature

Sequence: SEQ ID NO: 2:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tctgtaatca ctctgtgtcc 20
<212> Type : DNA
<211> Length : 20
   SequenceName : SEQ ID NO: 3
   SequenceDescription :

### Feature

Sequence: SEQ ID NO: 3:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agggacgata caacatgacc 20
<212> Type : DNA
<211> Length : 20
   SequenceName : SEQ ID NO: 4
   SequenceDescription :

### Feature

Sequence: SEQ ID NO: 4:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctattcagtc tctggaaacc 20
<212> Type : DNA
<211> Length : 20
   SequenceName : SEQ ID NO: 5
   SequenceDescription :

### Feature

Sequence: SEQ ID NO: 5:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atacatcaca ggggataacc 20
<212> Type : DNA
<211> Length : 20
   SequenceName : SEQ ID NO: 6
   SequenceDescription :

### Feature

Sequence: SEQ ID NO: 6:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgtagccaca acaacattgc 20
<212> Type : DNA
<211> Length : 20
   SequenceName : SEQ ID NO: 7
   SequenceDescription :

### Feature

Sequence: SEQ ID NO: 7:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aaagttacaa acgaagtggc 20
<212> Type : DNA
<211> Length : 20
   SequenceName : SEQ ID NO: 8
   SequenceDescription :

### Feature

Sequence: SEQ ID NO: 8:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcacttacac agacagctcc 20
<212> Type : DNA
<211> Length : 20
   SequenceName : SEQ ID NO: 9
   SequenceDescription :

### Feature

Sequence: SEQ ID NO: 9:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tatggacatg aaacaagacc 20
<212> Type : DNA
<211> Length : 20
   SequenceName : SEQ ID NO: 10
   SequenceDescription :

### Feature

Sequence: SEQ ID NO: 10:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcaactatac aaactattcc 20
<212> Type : DNA
<211> Length : 20
   SequenceName : SEQ ID NO: 11
   SequenceDescription :

### Feature

Sequence: SEQ ID NO: 11:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gttttcttgc tactcatacg 20
<212> Type : DNA
<211> Length : 20
   SequenceName : SEQ ID NO: 12
   SequenceDescription :

### Feature

Sequence: SEQ ID NO: 12:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgcacgtact ctgtcagtcg 20
<212> Type : DNA
<211> Length : 20
   SequenceName : SEQ ID NO: 13
   SequenceDescription :

### Feature

Sequence: SEQ ID NO: 13:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggatatgaga agcagaaagg 20
<212> Type : DNA
<211> Length : 20
   SequenceName : SEQ ID NO: 14
   SequenceDescription :

### Feature

Sequence: SEQ ID NO: 14:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggatatgaga agcagaaagg 20
<212> Type : DNA
<211> Length : 20
   SequenceName : SEQ ID NO: 15
   SequenceDescription :

### Feature

Sequence: SEQ ID NO: 15:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggytttgagg ctgaattta 19
<212> Type : DNA
<211> Length : 19
   SequenceName : SEQ ID NO: 16
   SequenceDescription :

### Feature

Sequence: SEQ ID NO: 16:
<221> FeatureKey : primer_bind
<222> LocationFrom : 1
<222> LocationTo : 19
   Other Information :
   CDSJoin : No

### Sequence

<213> OrganismName : Artificial Sequence
<400> PreSequenceString :
   gtccaatatc ctggagaagg 20
<212> Type : DNA
<211> Length : 20
   SequenceName : SEQ ID NO: 17
   SequenceDescription :

### Feature

Sequence: SEQ ID NO: 17:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aaccacttct ttccacttgg 20
<212> Type : DNA
<211> Length : 20
   SequenceName : SEQ ID NO: 18
   SequenceDescription :

### Feature

Sequence: SEQ ID NO: 18:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aatgcaatta tacagtgagc 20
<212> Type : DNA
<211> Length : 20
   SequenceName : SEQ ID NO: 19
   SequenceDescription :

### Feature

Sequence: SEQ ID NO: 19:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgagaacaca aagtcgaacg 20
<212> Type : DNA
<211> Length : 20
   SequenceName : SEQ ID NO: 20
   SequenceDescription :

### Sequence

<213> OrganismName : Artificial Sequence
<400> PreSequenceString :
   tcttaattgt acttatcagg 20
<212> Type : DNA
<211> Length : 20
   SequenceName : SEQ ID NO: 21
   SequenceDescription :

### Feature

Sequence: SEQ ID NO: 21:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcaatcaagc cagaaggagc 20
<212> Type : DNA
<211> Length : 20
   SequenceName : SEQ ID NO: 22
   SequenceDescription :

### Feature

Sequence: SEQ ID NO: 22:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcagtgttcc agagggagcc 20
<212> Type : DNA
<211> Length : 20
   SequenceName : SEQ ID NO: 23
   SequenceDescription :

### Feature

Sequence: SEQ ID NO: 23:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atggaaggtt tacagcacag 20
<212> Type : DNA
<211> Length : 20
   SequenceName : SEQ ID NO: 24
   SequenceDescription :

### Feature

Sequence: SEQ ID NO: 24:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   accctgagtg tccaggaggg 20
<212> Type : DNA
<211> Length : 20
   SequenceName : SEQ ID NO: 25
   SequenceDescription :

### Feature

Sequence: SEQ ID NO: 25:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ttatagacat tcgttcaaat 20
<212> Type : DNA
<211> Length : 20
   SequenceName : SEQ ID NO: 26
   SequenceDescription :

### Feature

Sequence: SEQ ID NO: 26:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tggactgcac atatgacacc 20
<212> Type : DNA
<211> Length : 20
   SequenceName : SEQ ID NO: 27
   SequenceDescription :

### Feature

Sequence: SEQ ID NO: 27:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cagcaaaatg caacagaagg 20
<212> Type : DNA
<211> Length : 20
   SequenceName : SEQ ID NO: 28
   SequenceDescription :

### Feature

Sequence: SEQ ID NO: 28:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agctgaagtg caactattcc 20
<212> Type : DNA
<211> Length : 20
   SequenceName : SEQ ID NO: 29
   SequenceDescription :

### Feature

Sequence: SEQ ID NO: 29:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tctagagaga gcatcaaagg 20
<212> Type : DNA
<211> Length : 20
   SequenceName : SEQ ID NO: 30
   SequenceDescription :

### Feature

Sequence: SEQ ID NO: 30:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aatgccacca tgaactgcag 20
<212> Type : DNA
<211> Length : 20
   SequenceName : SEQ ID NO: 31
   SequenceDescription :

### Feature

Sequence: SEQ ID NO: 31:
<221> FeatureKey : primer_bind
<222> LocationFrom : 1

<222> LocationTo : 20
   Other Information :
   CDSJoin : No

### Sequence

<213> OrganismName : Artificial Sequence
<400> PreSequenceString :
   gaaagagaga aacacagtgg 20
<212> Type : DNA
<211> Length : 20
   SequenceName : SEQ ID NO: 32
   SequenceDescription :

### Feature

Sequence: SEQ ID NO: 32:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gctctgacat taaactgcac 20
<212> Type : DNA
<211> Length : 20
   SequenceName : SEQ ID NO: 33
   SequenceDescription :

### Feature

Sequence: SEQ ID NO: 33:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   caggagacgg acagcagagg 20
<212> Type : DNA
<211> Length : 20
   SequenceName : SEQ ID NO: 34
   SequenceDescription :

### Feature

Sequence: SEQ ID NO: 34:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atgtgacctt ggactgtgtg 20
<212> Type : DNA
<211> Length : 20
   SequenceName : SEQ ID NO: 35
   SequenceDescription :

### Feature

Sequence: SEQ ID NO: 35:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gagcaaaatg aaataagtgg 20
<212> Type : DNA
<211> Length : 20
   SequenceName : SEQ ID NO: 36
   SequenceDescription :

### Feature

Sequence: SEQ ID NO: 36:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   actgcagtta cacagtcagc 20
<212> Type : DNA
<211> Length : 20
   SequenceName : SEQ ID NO: 37
   SequenceDescription :

### Feature

Sequence: SEQ ID NO: 37:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agaaagaaag gctaaaagcc 20
<212> Type : DNA
<211> Length : 20
   SequenceName : SEQ ID NO: 38
   SequenceDescription :

### Feature

Sequence: SEQ ID NO: 38:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   actgcagttt cactgatagc 20
<212> Type : DNA
<211> Length : 20
   SequenceName : SEQ ID NO: 39
   SequenceDescription :

### Feature

Sequence: SEQ ID NO: 39:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   caagtggaag acttaatgcc 20
<212> Type : DNA
<211> Length : 20
   SequenceName : SEQ ID NO: 40
   SequenceDescription :

### Feature

Sequence: SEQ ID NO: 40:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gggagccaat tccacgctgc 20
<212> Type : DNA
<211> Length : 20
   SequenceName : SEQ ID NO: 41
   SequenceDescription :

### Feature

Sequence: SEQ ID NO: 41:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atggaagatt aagcgccacg 20
<212> Type : DNA
<211> Length : 20
   SequenceName : SEQ ID NO: 42
   SequenceDescription :

### Feature

Sequence: SEQ ID NO: 42:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atttcaatta taaactgtgc 20
<212> Type : DNA
<211> Length : 20
   SequenceName : SEQ ID NO: 43
   SequenceDescription :

### Feature

Sequence: SEQ ID NO: 43:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aaggaagatt cacaatctcc 20
<212> Type : DNA
<211> Length : 20
   SequenceName : SEQ ID NO: 44
   SequenceDescription :

### Feature

Sequence: SEQ ID NO: 44:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcaccaattt cacctgcagc 20
<212> Type : DNA
<211> Length : 20
   SequenceName : SEQ ID NO: 45
   SequenceDescription :

### Feature

Sequence: SEQ ID NO: 45:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aggacgaata agtgccactc 20
<212> Type : DNA
<211> Length : 20
   SequenceName : SEQ ID NO: 46
   SequenceDescription :

### Feature

Sequence: SEQ ID NO: 46:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcaccacgta ctgcaattcc 20
<212> Type : DNA
<211> Length : 20
   SequenceName : SEQ ID NO: 47
   SequenceDescription :

### Feature

Sequence: SEQ ID NO: 47:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agactgacat ttcagtttgg 20
<212> Type : DNA
<211> Length : 20
   SequenceName : SEQ ID NO: 48
   SequenceDescription :

### Feature

Sequence: SEQ ID NO: 48:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcgacagatt cmctcccagg 20
<212> Type : DNA
<211> Length : 20
   SequenceName : SEQ ID NO: 49
   SequenceDescription :

### Feature

Sequence: SEQ ID NO: 49:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtccagyacc ttgatcctgc 20
<212> Type : DNA
<211> Length : 20
   SequenceName : SEQ ID NO: 50
   SequenceDescription :

### Feature

Sequence: SEQ ID NO: 50:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cctcaagtgt tttttccagc 20
<212> Type : DNA
<211> Length : 20
   SequenceName : SEQ ID NO: 51
   SequenceDescription :

### Feature

Sequence: SEQ ID NO: 51:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtgacagtag ttacgggtgg 20
<212> Type : DNA
<211> Length : 20
   SequenceName : SEQ ID NO: 52
   SequenceDescription :

### Feature

Sequence: SEQ ID NO: 52:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cagcatgttt gattatttcc 20
<212> Type : DNA
<211> Length : 20
   SequenceName : SEQ ID NO: 53
   SequenceDescription :

### Feature

Sequence: SEQ ID NO: 53:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctccaaggct ttatattctg 20
<212> Type : DNA
<211> Length : 20
   SequenceName : SEQ ID NO: 54
   SequenceDescription :

### Feature

Sequence: SEQ ID NO: 54:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctccaaggct ttatattctg 20
<212> Type : DNA
<211> Length : 20
   SequenceName : SEQ ID NO: 55
   SequenceDescription :

### Feature

Sequence: SEQ ID NO: 55:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atgatattac tgaagggtgg 20
<212> Type : DNA
<211> Length : 20
   SequenceName : SEQ ID NO: 56
   SequenceDescription :

### Feature

Sequence: SEQ ID NO: 56:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   actgcacgtc atcaaagacg 20
<212> Type : DNA
<211> Length : 20
   SequenceName : SEQ ID NO: 57
   SequenceDescription :

### Feature

Sequence: SEQ ID NO: 57:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ttgatgatgc tacagaaagg 20
<212> Type : DNA
<211> Length : 20
   SequenceName : SEQ ID NO: 58
   SequenceDescription :

### Feature

Sequence: SEQ ID NO: 58:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgaactgcac ttcttcaagc 20
<212> Type : DNA
<211> Length : 20
   SequenceName : SEQ ID NO: 59
   SequenceDescription :

### Feature

Sequence: SEQ ID NO: 59:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cttgatagcc ttatataagg 20
<212> Type : DNA
<211> Length : 20
   SequenceName : SEQ ID NO: 60
   SequenceDescription :

### Feature

Sequence: SEQ ID NO: 60:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcaattgcag ttatgaagtg 20
<212> Type : DNA
<211> Length : 20
   SequenceName : SEQ ID NO: 61
   SequenceDescription :

### Feature

Sequence: SEQ ID NO: 61:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tttatgctaa cttcaagtgg 20
<212> Type : DNA
<211> Length : 20
   SequenceName : SEQ ID NO: 62
   SequenceDescription :

### Feature

Sequence: SEQ ID NO: 62:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcacatatga caccagtgag 20
<212> Type : DNA
<211> Length : 20
   SequenceName : SEQ ID NO: 63
   SequenceDescription :

### Feature

Sequence: SEQ ID NO: 63:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcgccaagaa gcttataagc 20
<212> Type : DNA
<211> Length : 20
   SequenceName : SEQ ID NO: 64
   SequenceDescription :

### Feature

Sequence: SEQ ID NO: 64:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tctactgcaa ttattcaacc 20
<212> Type : DNA
<211> Length : 20
   SequenceName : SEQ ID NO: 65
   SequenceDescription :

### Feature

Sequence: SEQ ID NO: 65:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   caggagggac gattaatggc 20
<212> Type : DNA
<211> Length : 20
   SequenceName : SEQ ID NO: 66
   SequenceDescription :

### Feature

Sequence: SEQ ID NO: 66:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgaactgcac atacacatcc 20
<212> Type : DNA
<211> Length : 20
   SequenceName : SEQ ID NO: 67
   SequenceDescription :

### Feature

Sequence: SEQ ID NO: 67:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   acagcaaaaa cttcggaggc 20
<212> Type : DNA
<211> Length : 20
   SequenceName : SEQ ID NO: 68
   SequenceDescription :

### Feature

Sequence: SEQ ID NO: 68:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aactgcagtt actcggtagg 20
<212> Type : DNA
<211> Length : 20
   SequenceName : SEQ ID NO: 69
   SequenceDescription :

### Feature

Sequence: SEQ ID NO: 69:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aagcatggaa gattaattgc 20
<212> Type : DNA
<211> Length : 20
   SequenceName : SEQ ID NO: 70
   SequenceDescription :

### Feature

Sequence: SEQ ID NO: 70:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcagacagac ttgtcactgg 20
<212> Type : DNA
<211> Length : 20
   SequenceName : SEQ ID NO: 71
   SequenceDescription :

### Feature

Sequence: SEQ ID NO: 71:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agtctctcag ctggtacacg 20
<212> Type : DNA
<211> Length : 20
   SequenceName : SEQ ID NO: 72
   SequenceDescription :

### Feature

Sequence: SEQ ID NO: 72:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aatgaaacgt gagcatctgg 20
<212> Type : DNA
<211> Length : 20
   SequenceName : SEQ ID NO: 73
   SequenceDescription :

### Feature

Sequence: SEQ ID NO: 73:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cattgaaaac aagactgtgc 20
<212> Type : DNA
<211> Length : 20
   SequenceName : SEQ ID NO: 74
   SequenceDescription :

### Feature

Sequence: SEQ ID NO: 74:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtgtccccat ctctaatcac 20
<212> Type : DNA
<211> Length : 20
   SequenceName : SEQ ID NO: 75
   SequenceDescription :

### Feature

Sequence: SEQ ID NO: 75:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgaaatctca gagaagtctg 20
<212> Type : DNA
<211> Length : 20
   SequenceName : SEQ ID NO: 76
   SequenceDescription :

### Feature

Sequence: SEQ ID NO: 76:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tatgtattgg tataaacagg 20
<212> Type : DNA
<211> Length : 20
   SequenceName : SEQ ID NO: 77
   SequenceDescription :

### Feature

Sequence: SEQ ID NO: 77:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctctaagaaa tttctgaaga 20
<212> Type : DNA
<211> Length : 20
   SequenceName : SEQ ID NO: 78
   SequenceDescription :

### Sequence

<213> OrganismName : Artificial Sequence
<400> PreSequenceString :
   gtctttgaaa tgtgaacaac 20
<212> Type : DNA
<211> Length : 20
   SequenceName : SEQ ID NO: 79
   SequenceDescription :

### Feature

Sequence: SEQ ID NO: 79:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggagctcatg tttgtctaca 20
<212> Type : DNA
<211> Length : 20
   SequenceName : SEQ ID NO: 80
   SequenceDescription :

### Feature

Sequence: SEQ ID NO: 80:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gatcaaaacg agaggacagc 20
<212> Type : DNA
<211> Length : 20
   SequenceName : SEQ ID NO: 81
   SequenceDescription :

### Feature

Sequence: SEQ ID NO: 81:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   caggggcccc agtttatctt 20
<212> Type : DNA
<211> Length : 20 SequenceName : SEQ ID NO: 82 SequenceDescription :

### Feature

Sequence: SEQ ID NO: 82:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gaaacaragg aaacttccct 20
<212> Type : DNA
<211> Length : 20
   SequenceName : SEQ ID NO: 83
   SequenceDescription :

### Feature

Sequence: SEQ ID NO: 83:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtgtgcccag gatatgaacc 20
<212> Type : DNA
<211> Length : 20
   SequenceName : SEQ ID NO: 84
   SequenceDescription :

### Feature

Sequence: SEQ ID NO: 84:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   caggatatga gacataatgc 20
<212> Type : DNA
<211> Length : 20
   SequenceName : SEQ ID NO: 85
   SequenceDescription :

### Feature

Sequence: SEQ ID NO: 85:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggtatcgaca agacccaggc 20
<212> Type : DNA
<211> Length : 20
   SequenceName : SEQ ID NO: 86
   SequenceDescription :

### Feature

Sequence: SEQ ID NO: 86:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tagacaagat ctaggactgg 20
<212> Type : DNA
<211> Length : 20
   SequenceName : SEQ ID NO: 87
   SequenceDescription :

### Feature

Sequence: SEQ ID NO: 87:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctcaggtgtg atccaatttc 20
<212> Type : DNA
<211> Length : 20
   SequenceName : SEQ ID NO: 88
   SequenceDescription :

### Feature

Sequence: SEQ ID NO: 88:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tctaatttac ttccaaggca 20
<212> Type : DNA
<211> Length : 20
   SequenceName : SEQ ID NO: 89
   SequenceDescription :

### Feature

Sequence: SEQ ID NO: 89:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcccagagtg atgctcaacg 20
<212> Type : DNA
<211> Length : 20
   SequenceName : SEQ ID NO: 90
   SequenceDescription :

### Feature

Sequence: SEQ ID NO: 90:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   acttacttca attatgaagc 20
<212> Type : DNA
<211> Length : 20
   SequenceName : SEQ ID NO: 91
   SequenceDescription :

### Feature

Sequence: SEQ ID NO: 91:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequencestring :
   ccagaatgaa gctcaactag 20
<212> Type : DNA
<211> Length : 20
   SequenceName : SEQ ID NO: 92
   SequenceDescription :

### Feature

Sequence: SEQ ID NO: 92:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gagacctctc tgtgtactgg 20
<212> Type : DNA
<211> Length : 20
   SequenceName : SEQ ID NO: 93
   SequenceDescription :

### Feature

Sequence: SEQ ID NO: 93:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctcattcagt attataatgg 20
<212> Type : DNA
<211> Length : 20
   SequenceName : SEQ ID NO: 94
   SequenceDescription :

### Feature

Sequence: SEQ ID NO: 94:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggaatcaccc agagcccaag 20
<212> Type : DNA
<211> Length : 20
   SequenceName : SEQ ID NO: 95
   SequenceDescription :

### Feature

Sequence: SEQ ID NO: 95:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gacatgggct gaggctgatc 20
<212> Type : DNA
<211> Length : 20
   SequenceName : SEQ ID NO: 96
   SequenceDescription :

### Feature

Sequence: SEQ ID NO: 96:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cctaaggatc gattttctgc 20
<212> Type : DNA
<211> Length : 20
   SequenceName : SEQ ID NO: 97
   SequenceDescription :

### Feature

Sequence: SEQ ID NO: 97:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   actctcaaga tccagcctgc 20
<212> Type : DNA
<211> Length : 20
   SequenceName : SEQ ID NO: 98
   SequenceDescription :

### Feature

Sequence: SEQ ID NO: 98:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aggtgacaga gatgggacaa 20
<212> Type : DNA
<211> Length : 20
   SequenceName : SEQ ID NO: 99
   SequenceDescription :

### Feature

Sequence: SEQ ID NO: 99:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgcagggact ggaattgctg 20
<212> Type : DNA
<211> Length : 20
   SequenceName : SEQ ID NO: 100
   SequenceDescription :

### Feature

Sequence: SEQ ID NO: 100:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtacagacag accatgatgc 20
<212> Type : DNA
<211> Length : 20
   SequenceName : SEQ ID NO: 101
   SequenceDescription :

### Feature

Sequence: SEQ ID NO: 101:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctatcctatc cctagacacg 20
<212> Type : DNA
<211> Length : 20
   SequenceName : SEQ ID NO: 102
   SequenceDescription :

### Feature

Sequence: SEQ ID NO: 102:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aagatgcaga gcgataaagg 20
<212> Type : DNA
<211> Length : 20
   SequenceName : SEQ ID NO: 103
   SequenceDescription :

### Feature

Sequence: SEQ ID NO: 103:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agatgtgacc caatttctgg 20
<212> Type : DNA
<211> Length : 20
   SequenceName : SEQ ID NO: 104
   SequenceDescription :

### Feature

Sequence: SEQ ID NO: 104:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agtctaaaca ggatgagtcc 20
<212> Type : DNA
<211> Length : 20
   SequenceName : SEQ ID NO: 105
   SequenceDescription :

### Feature

Sequence: SEQ ID NO: 105:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcagactttg aaccataacg 20
<212> Type : DNA
<211> Length : 20
   SequenceName : SEQ ID NO: 106
   SequenceDescription :

### Feature

Sequence: SEQ ID NO: 106:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aaagatttta acaatgaagc 20
<212> Type : DNA
<211> Length : 20
   SequenceName : SEQ ID NO: 107
   SequenceDescription :

### Feature

Sequence: SEQ ID NO: 107:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tattgtgccc caataaaagg 20
<212> Type : DNA
<211> Length : 20
   SequenceName : SEQ ID NO: 108
   SequenceDescription :

### Feature

Sequence: SEQ ID NO: 108:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aatgtctttg atgaaacagg 20
<212> Type : DNA
<211> Length : 20
   SequenceName : SEQ ID NO: 109
   SequenceDescription :

### Feature

Sequence: SEQ ID NO: 109:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atccatcttc tggtcacatg 20
<212> Type : DNA
<211> Length : 20
   SequenceName : SEQ ID NO: 110
   SequenceDescription :

### Feature

Sequence: SEQ ID NO: 110:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aacattgcag ttgattcagg 20
<212> Type : DNA
<211> Length : 20
   SequenceName : SEQ ID NO: 111
   SequenceDescription :

### Feature

Sequence: SEQ ID NO: 111:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcagcccaat gaaaggacac 20
<212> Type : DNA
<211> Length : 20
   SequenceName : SEQ ID NO: 112
   SequenceDescription :

### Feature

Sequence: SEQ ID NO: 112:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aatatcatag atgagtcagg 20
<212> Type : DNA
<211> Length : 20
   SequenceName : SEQ ID NO: 113
   SequenceDescription :

### Feature

Sequence: SEQ ID NO: 113:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgaacagaat ttgaaccacg 20
<212> Type : DNA
<211> Length : 20
   SequenceName : SEQ ID NO: 114
   SequenceDescription :

### Feature

Sequence: SEQ ID NO: 114:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tttcagaaag gagatatagc 20
<212> Type : DNA
<211> Length : 20
   SequenceName : SEQ ID NO: 115
   SequenceDescription :

### Feature

Sequence: SEQ ID NO: 115:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcgagtgccg ttccctggac 20
<212> Type : DNA
<211> Length : 20
   SequenceName : SEQ ID NO: 116
   SequenceDescription :

### Feature

Sequence: SEQ ID NO: 116:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gatggcaact tccaatgagg 20
<212> Type : DNA
<211> Length : 20
   SequenceName : SEQ ID NO: 117
   SequenceDescription :

### Feature

Sequence: SEQ ID NO: 117:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcaaagatgg attgtgttcc 20
<212> Type : DNA
<211> Length : 20
   SequenceName : SEQ ID NO: 118
   SequenceDescription :

### Feature

Sequence: SEQ ID NO: 118:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cgctggaaga agagctcaag 20
<212> Type : DNA
<211> Length : 20
   SequenceName : SEQ ID NO: 119
   SequenceDescription :

### Feature

Sequence: SEQ ID NO: 119:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   catttggtca aaggaaaagg 20
<212> Type : DNA
<211> Length : 20
   SequenceName : SEQ ID NO: 120
   SequenceDescription :

### Feature

Sequence: SEQ ID NO: 120:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gaatgaacaa gttcttcaag 20
<212> Type : DNA
<211> Length : 20
   SequenceName : SEQ ID NO: 121
   SequenceDescription :

### Feature

Sequence: SEQ ID NO: 121:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atgctggaat gttctcagac 20
<212> Type : DNA
<211> Length : 20
   SequenceName : SEQ ID NO: 122
   SequenceDescription :

### Feature

Sequence: SEQ ID NO: 122:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtcaaagata taaacaaagg 20
<212> Type : DNA
<211> Length : 20
   SequenceName : SEQ ID NO: 123
   SequenceDescription :

### Feature

Sequence: SEQ ID NO: 123:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctctggaatg ttctcaaacc 20
<212> Type : DNA
<211> Length : 20
   SequenceName : SEQ ID NO: 124
   SequenceDescription :

### Feature

Sequence: SEQ ID NO: 124:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   taattccaca gagaagggag 20
<212> Type : DNA
<211> Length : 20
   SequenceName : SEQ ID NO: 125
   SequenceDescription :

### Feature

Sequence: SEQ ID NO: 125:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cccagaatat gaatcatgtt 20
<212> Type : DNA
<211> Length : 20
   SequenceName : SEQ ID NO: 126
   SequenceDescription :

### Feature

Sequence: SEQ ID NO: 126:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   attcacctgg cactgggagc 20
<212> Type : DNA
<211> Length : 20 SequenceName : SEQ ID NO: 127 SequenceDescription :

### Feature

Sequence: SEQ ID NO: 127:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ttgttctcag aatatgaacc 20
<212> Type : DNA
<211> Length : 20
   SequenceName : SEQ ID NO: 128
   SequenceDescription :

### Feature

Sequence: SEQ ID NO: 128:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgaggtgact gataagggag 20
<212> Type : DNA
<211> Length : 20
   SequenceName : SEQ ID NO: 129
   SequenceDescription :

### Feature

Sequence: SEQ ID NO: 129:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atgtgtccag gatatggacc 20
<212> Type : DNA
<211> Length : 20
   SequenceName : SEQ ID NO: 130
   SequenceDescription :

### Feature

Sequence: SEQ ID NO: 130:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aaaaggagat attcctgagg 20
<212> Type : DNA
<211> Length : 20
   SequenceName : SEQ ID NO: 131
   SequenceDescription :

### Feature

Sequence: SEQ ID NO: 131:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcaccatgat gttctggtac 20
<212> Type : DNA
<211> Length : 20
   SequenceName : SEQ ID NO: 132
   SequenceDescription :

### Feature

Sequence: SEQ ID NO: 132:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctggacagag cctgacactg 20
<212> Type : DNA
<211> Length : 20
   SequenceName : SEQ ID NO: 133
   SequenceDescription :

### Feature

Sequence: SEQ ID NO: 133:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgtggaggga acatcaaacc 20
<212> Type : DNA
<211> Length : 20
   SequenceName : SEQ ID NO: 134
   SequenceDescription :

### Feature

Sequence: SEQ ID NO: 134:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ttctactccg ttggtattgg 20
<212> Type : DNA
<211> Length : 20
   SequenceName : SEQ ID NO: 135
   SequenceDescription :

### Feature

Sequence: SEQ ID NO: 135:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtgtggcctt ttgggtgtgg 20
<212> Type : DNA
<211> Length : 20
   SequenceName : SEQ ID NO: 136
   SequenceDescription :

### Feature

Sequence: SEQ ID NO: 136:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tctgatggct caaacacagc 20
<212> Type : DNA
<211> Length : 20
   SequenceName : SEQ ID NO: 137
   SequenceDescription :

### Feature

Sequence: SEQ ID NO: 137:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgtatgaaac aagttggtgg 20
<212> Type : DNA
<211> Length : 20
   SequenceName : SEQ ID NO: 138
   SequenceDescription :

### Feature

Sequence: SEQ ID NO: 138:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cagaatgcaa aaagtggtcg 20
<212> Type : DNA
<211> Length : 20
   SequenceName : SEQ ID NO: 139
   SequenceDescription :

### Feature

Sequence: SEQ ID NO: 139:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atgaaaggag aagcgatcgg 20
<212> Type : DNA
<211> Length : 20
   SequenceName : SEQ ID NO: 140
   SequenceDescription :

### Feature

Sequence: SEQ ID NO: 140:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tggtttcaaa gacaatttcc 20
<212> Type : DNA
<211> Length : 20
   SequenceName : SEQ ID NO: 141
   SequenceDescription :

### Feature

Sequence: SEQ ID NO: 141:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gacactgtat attcaaatcc 20
<212> Type : DNA
<211> Length : 20
   SequenceName : SEQ ID NO: 142
   SequenceDescription :

### Feature

Sequence: SEQ ID NO: 142:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcagatttta ctcaaggacg 20
<212> Type : DNA
<211> Length : 20
   SequenceName : SEQ ID NO: 143
   SequenceDescription :

### Feature

Sequence: SEQ ID NO: 143:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agacaagcga catttgttcc 20
<212> Type : DNA
<211> Length : 20
   SequenceName : SEQ ID NO: 144
   SequenceDescription :

### Feature

Sequence: SEQ ID NO: 144:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   acggatggtt tggtatgagg 20
<212> Type : DNA
<211> Length : 20
   SequenceName : SEQ ID NO: 145
   SequenceDescription :

### Feature

Sequence: SEQ ID NO: 145:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gggtcatctg ctgaaatcac 20
<212> Type : DNA
<211> Length : 20
   SequenceName : SEQ ID NO: 146
   SequenceDescription :

### Feature

Sequence: SEQ ID NO: 146:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aggaggggaa ggccccacag 20
<212> Type : DNA
<211> Length : 20
   SequenceName : SEQ ID NO: 147
   SequenceDescription :

### Feature

Sequence: SEQ ID NO: 147:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gggtcatcag ctgtaatcac 20
<212> Type : DNA
<211> Length : 20
   SequenceName : SEQ ID NO: 148
   SequenceDescription :

### Feature

Sequence: SEQ ID NO: 148:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aggaggggaa gaccccacag 20
<212> Type : DNA
<211> Length : 20
   SequenceName : SEQ ID NO: 149
   SequenceDescription :

### Feature

Sequence: SEQ ID NO: 149:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agcccgcctg gaatgtgtgg 20
<212> Type : DNA
<211> Length : 20
   SequenceName : SEQ ID NO: 150
   SequenceDescription :

### Feature

Sequence: SEQ ID NO: 150:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcactgtcag aaaggaatcc 20
<212> Type : DNA
<211> Length : 20
   SequenceName : SEQ ID NO: 151
   SequenceDescription :

### Feature

Sequence: SEQ ID NO: 151:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aagaaaagta ttgacatacc 20
<212> Type : DNA
<211> Length : 20
   SequenceName : SEQ ID NO: 152
   SequenceDescription :

### Feature

Sequence: SEQ ID NO: 152:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atattgtctc aacaaaatcc 20
<212> Type : DNA
<211> Length : 20
   SequenceName : SEQ ID NO: 153
   SequenceDescription :

### Feature

Sequence: SEQ ID NO: 153:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agagtgccca catatcttgg 20
<212> Type : DNA
<211> Length : 20
   SequenceName : SEQ ID NO: 154
   SequenceDescription :

### Feature

Sequence: SEQ ID NO: 154:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gctcaagatt gctcaggtgg 20
<212> Type : DNA
<211> Length : 20
   SequenceName : SEQ ID NO: 155
   SequenceDescription :

### Feature

Sequence: SEQ ID NO: 155:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggatcccaga atcgtgttgc 20
<212> Type : DNA
<211> Length : 20
   SequenceName : SEQ ID NO: 156
   SequenceDescription :

### Feature

Sequence: SEQ ID NO: 156:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggtatgttcc agccttctgg 20
<212> Type : DNA
<211> Length : 20
   SequenceName : SEQ ID NO: 157
   SequenceDescription :

### Feature

Sequence: SEQ ID NO: 157:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agtgaaggtc tcctgcaagg 20
<212> Type : DNA
<211> Length : 20
   SequenceName : SEQ ID NO: 158
   SequenceDescription :

### Feature

Sequence: SEQ ID NO: 158:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agtgaaggtt tcctgcaagg 20
<212> Type : DNA
<211> Length : 20
   SequenceName : SEQ ID NO: 159
   SequenceDescription :

### Feature

Sequence: SEQ ID NO: 159:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agttccaggg cagagtcac 19
<212> Type : DNA
<211> Length : 19
   SequenceName : SEQ ID NO: 160
   SequenceDescription :

### Feature

Sequence: SEQ ID NO: 160:
<221> FeatureKey : primer_bind
<222> LocationFrom : 1
<222> LocationTo : 19
   Other Information :
   CDSJoin : No

### Sequence

<213> OrganismName : Artificial Sequence
<400> PreSequenceString :
   agtttcaggg cagggtcac 19
<212> Type : DNA
<211> Length : 19
   SequenceName : SEQ ID NO: 161
   SequenceDescription :

### Feature

Sequence: SEQ ID NO: 161:
<221> FeatureKey : primer_bind
<222> LocationFrom : 1
<222> LocationTo : 19
   Other Information :
   CDSJoin : No

### Sequence

<213> OrganismName : Artificial Sequence
<400> PreSequenceString :
   agttccagga aagagtcac 19
<212> Type : DNA
<211> Length : 19
   SequenceName : SEQ ID NO: 162
   SequenceDescription :

### Feature

Sequence: SEQ ID NO: 162:
<221> FeatureKey : primer_bind
<222> LocationFrom : 1
<222> LocationTo : 19
   Other Information :
   CDSJoin : No

### Sequence

<213> OrganismName : Artificial Sequence
<400> PreSequenceString :
   aattccagga cagagtcac 19
<212> Type : DNA
<211> Length : 19
   SequenceName : SEQ ID NO: 163
   SequenceDescription :

### Feature

Sequence: SEQ ID NO: 163:
<221> FeatureKey : primer_bind
<222> LocationFrom : 1
<222> LocationTo : 19
   Other Information :
   CDSJoin : No

### Sequence

<213> OrganismName : Artificial Sequence
<400> PreSequenceString :
   tctctgggtt ctcactcagc 20
<212> Type : DNA
<211> Length : 20
   SequenceName : SEQ ID NO: 164
   SequenceDescription :

### Feature

Sequence: SEQ ID NO: 164:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aaggccctgg agtggcttgc 20
<212> Type : DNA
<211> Length : 20
   SequenceName : SEQ ID NO: 165
   SequenceDescription :

### Feature

Sequence: SEQ ID NO: 165:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tccctgagac tctcctgtgc 20
<212> Type : DNA
<211> Length : 20
   SequenceName : SEQ ID NO: 166
   SequenceDescription :

### Feature

Sequence: SEQ ID NO: 166:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctctcctgtg cagcctctgg 20
<212> Type : DNA
<211> Length : 20
   SequenceName : SEQ ID NO: 167
   SequenceDescription :

### Feature

Sequence: SEQ ID NO: 167:
<221> FeatureKey : pririmer_bind
<222> LocationFrom : 1
<222> LocationTo : 20
   Other Information :
   CDSJoin : No

### Sequence

<213> OrganismName : Artificial Sequence
<400> PreSequenceString :
   ggtccctgag actctcctgt 20
<212> Type : DNA
<211> Length : 20
   SequenceName : SEQ ID NO: 168
   SequenceDescription :

### Feature

Sequence: SEQ ID NO: 168:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctgagactct cctgtgtagc 20
<212> Type : DNA
<211> Length : 20
   SequenceName : SEQ ID NO: 169
   SequenceDescription :

### Feature

Sequence: SEQ ID NO: 169:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctccagggaa ggggctgg 18
<212> Type : DNA
<211> Length : 18
   SequenceName : SEQ ID NO: 170
   SequenceDescription :

### Feature

Sequence: SEQ ID NO: 170:
<221> FeatureKey : primer_bind
<222> LocationFrom : 1
<222> LocationTo : 18
   Other Information :
   CDSJoin : No

### Sequence

<213> OrganismName Artificial Sequence
<400> PreSequenceString :
   ggctccaggc aaggggct 18
<212> Type : DNA
<211> Length : 18
   SequenceName : SEQ ID NO: 171
   SequenceDescription :

### Feature

Sequence: SEQ ID NO: 171:
<221> FeatureKey : primer_bind
<222> LocationFrom : 1
<222> LocationTo : 18
   Other Information :
   CDSJoin : No

### Sequence

<213> OrganismName : Artificial Sequence
<400> PreSequenceString :
   actgggtccg ccaggctcc 19
<212> Type : DNA
<211> Length : 19
   SequenceName : SEQ ID NO: 172
   SequenceDescription :

### Feature

Sequence: SEQ ID NO: 172:
<221> FeatureKey : primer_bind
<222> LocationFrom : 1
<222> LocationTo : 19
   Other Information :
   CDSJoin : No

### Sequence

<213> OrganismName : Artificial Sequence
<400> PreSequenceString :
   gaaggggctg gagtgggt 18
<212> Type : DNA
<211> Length : 18
   SequenceName : SEQ ID NO: 173
   SequenceDescription :

### Feature

Sequence: SEQ ID NO: 173:
<221> FeatureKey : primer_bind
<222> LocationFrom : 1
<222> LocationTo : 18
   Other Information :
   CDSJoin : No

### Sequence

<213> OrganismName : Artificial Sequence
<400> PreSequenceString :
   aaaaggtctg gagtgggt 18
<212> Type : DNA
<211> Length : 18
   SequenceName : SEQ ID NO: 174
   SequenceDescription :

### Feature

Sequence: SEQ ID NO: 174:
<221> FeatureKey : primer_bind
<222> LocationFrom : 1
<222> LocationTo : 18
   Other Information :
   CDSJoin : No

### Sequence

<213> OrganismName : Artificial Sequence
<400> PreSequenceString :
   agaccctgtc cctcacctgc 20
<212> Type : DNA
<211> Length : 20
   SequenceName : SEQ ID NO: 175
   SequenceDescription :

### Feature

Sequence: SEQ ID NO: 175:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agggvctgga gtggattggg 20
<212> Type : DNA
<211> Length : 20
   SequenceName : SEQ ID NO: 176
   SequenceDescription :

### Feature

Sequence: SEQ ID NO: 176:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcgccagatg cccgggaaag 20
<212> Type : DNA
<211> Length : 20
   SequenceName : SEQ ID NO: 177
   SequenceDescription :

### Feature

Sequence: SEQ ID NO: 177:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggccasgtca ccatctcagc 20
<212> Type : DNA
<211> Length : 20
   SequenceName : SEQ ID NO: 178
   SequenceDescription :

### Feature

Sequence: SEQ ID NO: 178:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ccggggacag tgtctctagc 20
<212> Type : DNA
<211> Length : 20
   SequenceName : SEQ ID NO: 179
   SequenceDescription :

### Feature

Sequence: SEQ ID NO: 179:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gccttgagtg gctgggaagg 20
<212> Type : DNA
<211> Length : 20
   SequenceName : SEQ ID NO: 180
   SequenceDescription :

### Feature

Sequence: SEQ ID NO: 180:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtttcctgca aggcttctgg 20
<212> Type : DNA
<211> Length : 20
   SequenceName : SEQ ID NO: 181
   SequenceDescription :

### Feature

Sequence: SEQ ID NO: 181:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggcttgagtg gatgggatgg 20
<212> Type : DNA
<211> Length : 20
   SequenceName : SEQ ID NO: 182
   SequenceDescription :

### Feature

Sequence: SEQ ID NO: 182:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   acctgaggag acggtgacc 19
<212> Type : DNA
<211> Length : 19
   SequenceName : SEQ ID NO: 183
   SequenceDescription :

### Feature

Sequence: SEQ ID NO: 183:
<221> FeatureKey : primer_bind
<222> LocationFrom : 1
<222> LocationTo : 19
   Other Information :
   CDSJoin : No

### Sequence

<213> OrganismName : Artificial Sequence
<400> PreSequenceString :
   cagtgctgga agtattcagc 20
<212> Type : DNA
<211> Length : 20
   SequenceName : SEQ ID NO: 184
   SequenceDescription :

### Feature

Sequence: SEQ ID NO: 184:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agagatcgaa gtaccagtag 20
<212> Type : DNA
<211> Length : 20
   SequenceName : SEQ ID NO: 185
   SequenceDescription :

### Feature

Sequence: SEQ ID NO: 185:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ccccagatat caaaagcatc 20
<212> Type : DNA
<211> Length : 20
   SequenceName : SEQ ID NO: 186
   SequenceDescription :

### Feature

Sequence: SEQ ID NO: 186:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggccccagta gtcaaagtag 20
<212> Type : DNA
<211> Length : 20
   SequenceName : SEQ ID NO: 187
   SequenceDescription :

### Feature

Sequence: SEQ ID NO: 187:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString : 20
   cccaggggtc gaaccagttg
<212> Type : DNA
<211> Length : 20
   SequenceName : SEQ ID NO: 188
   SequenceDescription :

### Feature

Sequence: SEQ ID NO: 188:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cccagacgtc catgtagtag 20
<212> Type : DNA
<211> Length : 20
   SequenceName : SEQ ID NO: 189
   SequenceDescription :

### Feature

Sequence: SEQ ID NO: 189:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   taggagacag agtcaccatc 20
<212> Type : DNA
<211> Length : 20
   SequenceName : SEQ ID NO: 190
   SequenceDescription :

### Feature

Sequence: SEQ ID NO: 190:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ttcagygrca gtggatctgg 20
<212> Type : DNA
<211> Length : 20
   SequenceName : SEQ ID NO: 191
   SequenceDescription :

### Feature

Sequence: SEQ ID NO: 191:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggagagccgg cctccatctc 20
<212> Type : DNA
<211> Length : 20
   SequenceName : SEQ ID NO: 192
   SequenceDescription :

### Feature

Sequence: SEQ ID NO: 192:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tggtacctgc agaagccagg 20
<212> Type : DNA
<211> Length : 20
   SequenceName : SEQ ID NO: 193
   SequenceDescription :

### Feature

Sequence: SEQ ID NO: 193:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cttcagcaga ggccaggcca 20
<212> Type : DNA
<211> Length : 20
   SequenceName : SEQ ID NO: 194
   SequenceDescription :

### Feature

Sequence: SEQ ID NO: 194:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcctggtacc agcagaaacc 20
<212> Type : DNA
<211> Length : 20
   SequenceName : SEQ ID NO: 195
   SequenceDescription :

### Feature

Sequence: SEQ ID NO: 195:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gccaggttca gtggcagtgg 20
<212> Type : DNA
<211> Length : 20
   SequenceName : SEQ ID NO: 196
   SequenceDescription :

### Feature

Sequence: SEQ ID NO: 196:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcgaggttca gtggcagtgg 20
<212> Type : DNA
<211> Length : 20
   SequenceName : SEQ ID NO: 197
   SequenceDescription :

### Feature

Sequence: SEQ ID NO: 197:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gaccgattca gtggcagcgg 20
<212> Type : DNA
<211> Length : 20
   SequenceName : SEQ ID NO: 198
   SequenceDescription :

### Feature

Sequence: SEQ ID NO: 198:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ttcaactgct catcagatgg 20
<212> Type : DNA
<211> Length : 20
   SequenceName : SEQ ID NO: 199
   SequenceDescription :

### Feature

Sequence: SEQ ID NO: 199:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atgaagacag atggtgcagc 20
<212> Type : DNA
<211> Length : 20
   SequenceName : SEQ ID NO: 200
   SequenceDescription :

### Feature

Sequence: SEQ ID NO: 200:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gggcagaggg tcaccatctc 20
<212> Type : DNA
<211> Length : 20
   SequenceName : SEQ ID NO: 201
   SequenceDescription :

### Feature

Sequence: SEQ ID NO: 201:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggacagaagg tcaccatctc 20
<212> Type : DNA
<211> Length : 20
   SequenceName : SEQ ID NO: 202
   SequenceDescription :

### Feature

Sequence: SEQ ID NO: 202:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tggtaccagc agctcccagg 20
<212> Type : DNA
<211> Length : 20
   SequenceName : SEQ ID NO: 203
   SequenceDescription :

### Feature

Sequence: SEQ ID NO: 203:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tggtaccagc agcttccagg 20
<212> Type : DNA
<211> Length : 20
   SequenceName : SEQ ID NO: 204
   SequenceDescription :

### Feature

Sequence: SEQ ID NO: 204:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctgcactgga accagcagtg 20
<212> Type : DNA
<211> Length : 20
   SequenceName : SEQ ID NO: 205
   SequenceDescription :

### Feature

Sequence: SEQ ID NO: 205:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tctctggctc caagtctggc 20
<212> Type : DNA
<211> Length : 20
   SequenceName : SEQ ID NO: 206
   SequenceDescription :

### Feature

Sequence: SEQ ID NO: 206:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   accagcagaa gccaggccag 20
<212> Type : DNA
<211> Length : 20
   SequenceName : SEQ ID NO: 207
   SequenceDescription :

### Feature

Sequence: SEQ ID NO: 207:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gaagccagga caggcccctg 20
<212> Type : DNA
<211> Length : 20
   SequenceName : SEQ ID NO: 208
   SequenceDescription :

### Feature

Sequence: SEQ ID NO: 208:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctgagcgatt ctctggctcc 20
<212> Type : DNA
<211> Length : 20
   SequenceName : SEQ ID NO: 209
   SequenceDescription :

### Feature

Sequence: SEQ ID NO: 209:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ttctctgggt ccacctcagg 20
<212> Type : DNA
<211> Length : 20
   SequenceName : SEQ ID NO: 210
   SequenceDescription :

### Feature

Sequence: SEQ ID NO: 210:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ttctctggct ccagctcagg 20
<212> Type : DNA
<211> Length : 20
   SequenceName : SEQ ID NO: 211
   SequenceDescription :

### Feature

Sequence: SEQ ID NO: 211:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tcggtcaagc tcacctgcac 20
<212> Type : DNA
<211> Length : 20
   SequenceName : SEQ ID NO: 212
   SequenceDescription :

### Feature

Sequence: SEQ ID NO: 212:
<221> FeatureKey : primer_bind
<222> LocationFrom : 1
<222> LocationTo : 20
   Other Information :
   CDSJoin : No

### Sequence

<213> OrganismName : Artificial Sequence
<900> PreSequenceString :
   gggctgaccg ctacctcacc 20
<212> Type : DNA
<211> Length : 20
   SequenceName : SEQ ID NO: 213
   SequenceDescription :

### Feature

Sequence: SEQ ID NO: 213:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cagcctgtgc tgactcagcc 20
<212> Type : DNA
<211> Length : 20
   SequenceName : SEQ ID NO: 214
   SequenceDescription :

### Feature

Sequence: SEQ ID NO: 214:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ccagccgctt ctctggatcc 20
<212> Type : DNA
<211> Length : 20
   SequenceName : SEQ ID NO: 215
   SequenceDescription :

### Feature

Sequence: SEQ ID NO: 215:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ccatctcctg cacccgcagc 20
<212> Type : DNA
<211> Length : 20
   SequenceName : SEQ ID NO: 216
   SequenceDescription :

### Feature

Sequence: SEQ ID NO: 216:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tccccwggag ggacagtcac 20
<212> Type : DNA
<211> Length : 20
   SequenceName : SEQ ID NO: 217
   SequenceDescription :

### Feature

Sequence: SEQ ID NO: 217:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctcmcctgca ccctgagcag 20
<212> Type : DNA
<211> Length : 20
   SequenceName : SEQ ID NO: 218
   SequenceDescription :

### Feature

Sequence: SEQ ID NO: 218:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agaccgccac actcacctgc 20
<212> Type : DNA
<211> Length : 20
   SequenceName : SEQ ID NO: 219
   SequenceDescription :

### Feature

Sequence: SEQ ID NO: 219:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctgatcgstt ctctggctcc 20
<212> Type : DNA
<211> Length : 20
   SequenceName : SEQ ID NO: 220
   SequenceDescription :

### Feature

Sequence: SEQ ID NO: 220:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctgcccggtt ctcaggctcc 20
<212> Type : DNA
<211> Length : 20
   SequenceName : SEQ ID NO: 221
   SequenceDescription :

### Feature

Sequence: SEQ ID NO: 221:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   atccaggaag aggatgagag 20
<212> Type : DNA
<211> Length : 20
   SequenceName : SEQ ID NO: 222
   SequenceDescription :

### Feature

Sequence: SEQ ID NO: 222:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctccagcctg aggacgaggc 20
<212> Type : DNA
<211> Length : 20
   SequenceName : SEQ ID NO: 223
   SequenceDescription :

### Feature

Sequence: SEQ ID NO: 223:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gctcccgggt agaagtcact 20
<212> Type : DNA
<211> Length : 20
   SequenceName : SEQ ID NO: 224
   SequenceDescription :

### Feature

Sequence: SEQ ID NO: 224:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agtgtggcct tgttggcttg 20
<212> Type : DNA
<211> Length : 20
   SequenceName : SEQ ID NO: 225
   SequenceDescription :

### Feature

Sequence: SEQ ID NO: 225:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cccactccat gaggtatttc 20
<212> Type : DNA
<211> Length : 20
   SequenceName : SEQ ID NO: 226
   SequenceDescription :

### Feature

Sequence: SEQ ID NO: 226:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cctactccat gaggtatttc 20
<212> Type : DNA
<211> Length : 20
   SequenceName : SEQ ID NO: 227
   SequenceDescription :

### Feature

Sequence: SEQ ID NO: 227:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcggggagcc ccgcttcatc 20
<212> Type : DNA
<211> Length : 20
   SequenceName : SEQ ID NO: 228
   SequenceDescription :

### Feature

Sequence: SEQ ID NO: 228:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcgggaagcc ccgcttcatc 20
<212> Type : DNA
<211> Length : 20
   SequenceName : SEQ ID NO: 229
   SequenceDescription :

### Feature

Sequence: SEQ ID NO: 229:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtggagagcc ccgcttcatc 20
<212> Type : DNA
<211> Length : 20
   SequenceName : SEQ ID NO: 230
   SequenceDescription :

### Feature

Sequence: SEQ ID NO: 230:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcggaaagcc ccgcttcatc 20
<212> Type : DNA
<211> Length : 20
   SequenceName : SEQ ID NO: 231
   SequenceDescription :

### Feature

Sequence: SEQ ID NO: 231:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcggaaagcc ccacttcatc 20
<212> Type : DNA
<211> Length : 20
   SequenceName : SEQ ID NO: 232
   SequenceDescription :

### Feature

Sequence: SEQ ID NO: 232:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcgggaagcc ccacttcatc 20
<212> Type : DNA
<211> Length : 20
   SequenceName : SEQ ID NO: 233
   SequenceDescription :

### Feature

Sequence: SEQ ID NO: 233:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtgggctacg tggacgacac 20
<212> Type : DNA
<211> Length : 20
   SequenceName : SEQ ID NO: 234
   SequenceDescription :

### Feature

Sequence: SEQ ID NO: 234:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtgggctacg tggacggcac 20
<212> Type : DNA
<211> Length : 20
   SequenceName : SEQ ID NO: 235
   SequenceDescription :

### Feature

Sequence: SEQ ID NO: 235:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gttcgtgcgg ttcgacagcg 20
<212> Type : DNA
<211> Length : 20
   SequenceName : SEQ ID NO: 236
   SequenceDescription :

### Feature

Sequence: SEQ ID NO: 236:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gttcgtgcgg tttgacagcg 20
<212> Type : DNA
<211> Length : 20
   SequenceName : SEQ ID NO: 237
   SequenceDescription :

### Feature

Sequence: SEQ ID NO: 237:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gttcgtgagg ttcgacagcg 20
<212> Type : DNA
<211> Length : 20
   SequenceName : SEQ ID NO: 238
   SequenceDescription :

### Feature

Sequence: SEQ ID NO: 238:
<221> FeatureKey : primer_birid
<222> LocationFrom : 1
<222> LocationTo : 20
   Other Information :
   CDSJoin : No

### Sequence

<213> OrganismName : Artificial Sequence
<400> PreSequenceString :
   taatccttgc cgtcgtaggc 20
<212> Type : DNA
<211> Length : 20
   SequenceName : SEQ ID NO: 239
   SequenceDescription :

### Feature

Sequence: SEQ ID NO: 239:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   taatccttgc cgtcgtaagc 20
<212> Type : DNA
<211> Length : 20
   SequenceName : SEQ ID NO: 240
   SequenceDescription :

### Feature

Sequence: SEQ ID NO: 240:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   taatctttgc cgtcgtaggc 20
<212> Type : DNA
<211> Length : 20
   SequenceName : SEQ ID NO: 241
   SequenceDescription :

### Feature

Sequence: SEQ ID NO: 241:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggtcctcgtt cagggcgatg 20
<212> Type : DNA
<211> Length : 20
   SequenceName : SEQ ID NO: 242
   SequenceDescription :

### Feature

Sequence: SEQ ID NO: 242:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggtcctcttt cagggcgatg 20
<212> Type : DNA
<211> Length : 20
   SequenceName : SEQ ID NO: 243
   SequenceDescription :

### Feature

Sequence: SEQ ID NO: 243:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggtcctcgtt caaggcgatg 20
<212> Type : DNA
<211> Length : 20
   SequenceName : SEQ ID NO: 244
   SequenceDescription :

### Feature

Sequence: SEQ ID NO: 244:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gatcctcgtt cagggcgatg 20
<212> Type : DNA
<211> Length : 20
   SequenceName : SEQ ID NO: 245
   SequenceDescription :

### Feature

Sequence: SEQ ID NO: 245:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggtcctcatt cagggcgatg 20
<212> Type : DNA
<211> Length : 20
   SequenceName : SEQ ID NO: 246
   SequenceDescription :

### Feature

Sequence: SEQ ID NO: 296:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcctacgacg gcaaggatta 20
<212> Type : DNA
<211> Length : 20
   SequenceName : SEQ ID NO: 247
   SequenceDescription :

### Feature

Sequence: SEQ ID NO: 247:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcttacgacg gcaaggatta 20
<212> Type : DNA
<211> Length : 20
   SequenceName : SEQ ID NO: 248
   SequenceDescription :

### Feature

Sequence: SEQ ID NO: 248:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcctacgacg gcaaagatta 20
<212> Type : DNA
<211> Length : 20
   SequenceName : SEQ ID NO: 249
   SequenceDescription :

### Feature

Sequence: SEQ ID NO: 249:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   catcgccctg aacgaggacc 20
<212> Type : DNA
<211> Length : 20
   SequenceName : SEQ ID NO: 250
   SequenceDescription :

### Feature

Sequence: SEQ ID NO: 250:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   catcgccctg aaagaggacc 20
<212> Type : DNA
<211> Length : 20
   SequenceName : SEQ ID NO: 251
   SequenceDescription :

### Feature

Sequence: SEQ ID NO: 251:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   catcgccttg aacgaggacc 20
<212> Type : DNA
<211> Length : 20
   SequenceName : SEQ ID NO: 252
   SequenceDescription :

### Feature

Sequence: SEQ ID NO: 252:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   catcgccctg aacgaggatc 20
<212> Type : DNA
<211> Length : 20
   SequenceName : SEQ ID NO: 253
   SequenceDescription :

### Feature

Sequence: SEQ ID NO: 253:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   catcgccctg aatgaggacc 20
<212> Type : DNA
<211> Length : 20
   SequenceName : SEQ ID NO: 254
   SequenceDescription :

### Feature

Sequence: SEQ ID NO: 254:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggtatctgcg gagcccgtcc 20
<212> Type : DNA
<211> Length : 20
   SequenceName : SEQ ID NO: 255
   SequenceDescription :

### Feature

Sequence: SEQ ID NO: 255:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggtatctgcg gagccactcc 20
<212> Type : DNA
<211> Length : 20
   SequenceName : SEQ ID NO: 256
   SequenceDescription :

### Feature

Sequence: SEQ ID NO: 256:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggtgtctgcg gagccactcc 20
<212> Type : DNA
<211> Length : 20
   SequenceName : SEQ ID NO: 257
   SequenceDescription :

### Feature

Sequence: SEQ ID NO: 257:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggtatccgcg gagccactcc 20
<212> Type : DNA
<211> Length : 20
   SequenceName : SEQ ID NO: 258
   SequenceDescription :

### Feature

Sequence: SEQ ID NO: 258:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcagcgtctc cttcccgttc 20
<212> Type : DNA
<211> Length : 20
   SequenceName : SEQ ID NO: 259
   SequenceDescription :

### Feature

Sequence: SEQ ID NO: 259:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ccagcttgtc cttcccgttc 20
<212> Type : DNA
<211> Length : 20
   SequenceName : SEQ ID NO: 260
   SequenceDescription :

### Feature

Sequence: SEQ ID NO: 260:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ccagcgtgtc cttcccgttc 20
<212> Type : DNA
<211> Length : 20
   SequenceName : SEQ ID NO: 261
   SequenceDescription :

### Feature

Sequence: SEQ ID NO: 261:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcagcgtctc cttcccattc 20
<212> Type : DNA
<211> Length : 20
   SequenceName : SEQ ID NO: 262
   SequenceDescription :

### Feature

Sequence: SEQ ID NO: 262:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gcagcgtctc cttcckgttc 20
<212> Type : DNA
<211> Length : 20
   SequenceName : SEQ ID NO: 263
   SequenceDescription :

### Feature

Sequence: SEQ ID NO: 263:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgtcatttct tcaatgggac 20
<212> Type : DNA
<211> Length : 20
   SequenceName : SEQ ID NO: 264
   SequenceDescription :

### Feature

Sequence: SEQ ID NO: 264:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   agtgtcattt cttcaacggg 20
<212> Type : DNA
<211> Length : 20
   SequenceName : SEQ ID NO: 265
   SequenceDescription :

### Feature

Sequence: SEQ ID NO: 265:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtgttatttc ttcaatggga 20
<212> Type : DNA
<211> Length : 20
   SequenceName : SEQ ID NO: 266
   SequenceDescription :

### Feature

Sequence: SEQ ID NO: 266:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtgtcaattc ttcaatggga 20
<212> Type : DNA
<211> Length : 20
   SequenceName : SEQ ID NO: 267
   SequenceDescription :

### Feature

Sequence: SEQ ID NO: 267:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtgtcatttc ctcaatggga 20
<212> Type : DNA
<211> Length : 20
   SequenceName : SEQ ID NO: 268
   SequenceDescription :

### Feature

Sequence: SEQ ID NO: 268:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggagcgggtg cggttgctgg 20
<212> Type : DNA
<211> Length : 20
   SequenceName : SEQ ID NO: 269
   SequenceDescription :

### Feature

Sequence: SEQ ID NO: 269:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggagcgggtg cggtacctgg 20
<212> Type : DNA
<211> Length : 20
   SequenceName : SEQ ID NO: 270
   SequenceDescription :

### Feature

Sequence: SEQ ID NO: 270:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggagcgggtg cggttcctgg 20
<212> Type : DNA
<211> Length : 20
   SequenceName : SEQ ID NO: 271
   SequenceDescription :

### Feature

Sequence: SEQ ID NO: 271:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggagcgggtg cgattcctgg 20
<212> Type : DNA
<211> Length : 20
   SequenceName : SEQ ID NO: 272
   SequenceDescription :

### Feature

Sequence: SEQ ID NO: 272:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggagcgggtg cggtatctgc 20
<212> Type : DNA
<211> Length : 20
   SequenceName : SEQ ID NO: 273
   SequenceDescription :

### Feature

Sequence: SEQ ID NO: 273:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggagcgggtg cggttactgg 20
<212> Type : DNA
<211> Length : 20
   SequenceName : SEQ ID NO: 274
   SequenceDescription :

### Feature

Sequence: SEQ ID NO: 274:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   acatctataa ccaagaggag 20
<212> Type : DNA
<211> Length : 20
   SequenceName : SEQ ID NO: 275
   SequenceDescription :

### Feature

Sequence: SEQ ID NO: 275:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   acatccataa acgggaggag 20
<212> Type : DNA
<211> Length : 20
   SequenceName : SEQ ID NO: 276
   SequenceDescription :

### Feature

Sequence: SEQ ID NO: 276:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tataaccaag aggagtacgt 20
<212> Type : DNA
<211> Length : 20
   SequenceName : SEQ ID NO: 277
   SequenceDescription :

### Feature

Sequence: SEQ ID NO: 277:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aaccccgtag ttgtgtctgc 20
<212> Type : DNA
<211> Length : 20
   SequenceName : SEQ ID NO: 278
   SequenceDescription :

### Feature

Sequence: SEQ ID NO: 278:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aaccccgtag ttgtatctgc 20
<212> Type : DNA
<211> Length : 20
   SequenceName : SEQ ID NO: 279
   SequenceDescription :

### Feature

Sequence: SEQ ID NO: 279:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cgtaattgta tctgcagtag 20
<212> Type : DNA
<211> Length : 20
   SequenceName : SEQ ID NO: 280
   SequenceDescription :

### Feature

Sequence: SEQ ID NO: 280:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tagttgtcca cttcggcccg 20
<212> Type : DNA
<211> Length : 20
   SequenceName : SEQ ID NO: 281
   SequenceDescription :

### Feature

Sequence: SEQ ID NO: 281:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cgctgcactg tgaagctctc 20
<212> Type : DNA
<211> Length : 20
   SequenceName : SEQ ID NO: 282
   SequenceDescription :

### Feature

Sequence: SEQ ID NO: 282:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cgctgcaccg tgaagctctc 20
<212> Type : DNA
<211> Length : 20
   SequenceName : SEQ ID NO: 283
   SequenceDescription :

### Feature

Sequence: SEQ ID NO: 283:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cctgtggaac tgagagagcc 20
<212> Type : DNA
<211> Length : 20
   SequenceName : SEQ ID NO: 284
   SequenceDescription :

### Feature

Sequence: SEQ ID NO: 284:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   caacgtcctc atctgtttca 20
<212> Type : DNA
<211> Length : 20
   SequenceName : SEQ ID NO: 285
   SequenceDescription :

### Feature

Sequence: SEQ ID NO: 285:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctgctgcatt gcttttgcgc 20
<212> Type : DNA
<211> Length : 20
   SequenceName : SEQ ID NO: 286
   SequenceDescription :

### Feature

Sequence: SEQ ID NO: 286:
<221> FeatureKey : primer bind
<222> LocationFrom : 1
<222> LocationTo : 20
   Other Information :
   CDSJoin : No

### Sequence

<213> OrganismName : Artificial Sequence
<400> PreSequenceString :
   ttacagaggc cccctgcgtt 20
<212> Type : DNA
<211> Length : 20
   SequenceName : SEQ ID NO: 287
   SequenceDescription :

### Feature

Sequence: SEQ ID NO: 287:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gtccagggca gggccactcc 20
<212> Type : DNA
<211> Length : 20
   SequenceName : SEQ ID NO: 288
   SequenceDescription :

### Feature

Sequence: SEQ ID NO: 288:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aattacgtgt accagggacg 20
<212> Type : DNA
<211> Length : 20
   SequenceName : SEQ ID NO: 289
   SequenceDescription :

### Feature

Sequence: SEQ ID NO: 289:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aattaccttt tccagggacg 20
<212> Type : DNA
<211> Length : 20
   SequenceName : SEQ ID NO: 290
   SequenceDescription :

### Feature

Sequence: SEQ ID NO: 290:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aattacgtgt accagttacg 20
<212> Type : DNA
<211> Length : 20
   SequenceName : SEQ ID NO: 291
   SequenceDescription :

### Feature

Sequence: SEQ ID NO: 291:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aattacgcgt accagttacg 20
<212> Type : DNA
<211> Length : 20
   SequenceName : SEQ ID NO: 292
   SequenceDescription :

### Feature

Sequence: SEQ ID NO: 292:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cggcctcgtc cagctcgtag 20
<212> Type : DNA
<211> Length : 20
   SequenceName : SEQ ID NO: 293
   SequenceDescription :

### Feature

Sequence: SEQ ID NO: 293:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgggcccgcc cagctcgtag 20
<212> Type : DNA
<211> Length : 20
   SequenceName : SEQ ID NO: 294
   SequenceDescription :

### Feature

Sequence: SEQ ID NO: 294:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgggcccgac cagctcgtag 20
<212> Type : DNA
<211> Length : 20
   SequenceName : SEQ ID NO: 295
   SequenceDescription :

### Feature

Sequence: SEQ ID NO: 295:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggactcggcg ctgcagggtc 20
<212> Type : DNA
<211> Length : 20
   SequenceName : SEQ ID NO: 296
   SequenceDescription :

### Feature

Sequence: SEQ ID NO: 296:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aggagctggg gccatcaagg 20
<212> Type : DNA
<211> Length : 20
   SequenceName : SEQ ID NO: 297
   SequenceDescription :

### Feature

Sequence: SEQ ID NO: 297:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   gaccatgtgt caacttatgc 20
<212> Type : DNA
<211> Length : 20
   SequenceName : SEQ ID NO: 298
   SequenceDescription :

### Feature

Sequence: SEQ ID NO: 298:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctcaggggga tcgttggtgg 20
<212> Type : DNA
<211> Length : 20
   SequenceName : SEQ ID NO: 299
   SequenceDescription :

### Feature

Sequence: SEQ ID NO: 299:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ctcaggggga tcattggcgg 20
<212> Type : DNA
<211> Length : 20
   SequenceName : SEQ ID NO: 300
   SequenceDescription :

### Feature

Sequence: SEQ ID NO: 300:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   cagctccaca ggctccttgg 20
<212> Type : DNA
<211> Length : 20
   SequenceName : SEQ ID NO: 301
   SequenceDescription :

### Feature

Sequence: SEQ ID NO: 301:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   actctcccga ggatttcgtg 20
<212> Type : DNA
<211> Length : 20
   SequenceName : SEQ ID NO: 302
   SequenceDescription :

### Feature

Sequence: SEQ ID NO: 302:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tgtgctactt caccaacggg 20
<212> Type : DNA
<211> Length : 20
   SequenceName : SEQ ID NO: 303
   SequenceDescription :

### Feature

Sequence: SEQ ID NO: 303:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   acctcgtagt tgtgtctgca 20
<212> Type : DNA
<211> Length : 20
   SequenceName : SEQ ID NO: 304
   SequenceDescription :

### Feature

Sequence: SEQ ID NO: 304:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   aactggtagt tgtgtctgca 20
<212> Type : DNA
<211> Length : 20
   SequenceName : SEQ ID NO: 305
   SequenceDescription :

### Feature

Sequence: SEQ ID NO: 305:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   actctcctct gcaggatccc 20
<212> Type : DNA
<211> Length : 20
   SequenceName : SEQ ID NO: 306
   SequenceDescription,:

### Feature

Sequence: SEQ ID NO: 306:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   actcgccgct gcaaggtcgt 20
<212> Type : DNA
<211> Length : 20
   SequenceName : SEQ ID NO: 307
   SequenceDescription :

### Feature

Sequence: SEQ ID NO: 307:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   actctcctct gcaagatccc 20
<212> Type : DNA
<211> Length : 20
   SequenceName : SEQ ID NO: 308
   SequenceDescription :

### Feature

Sequence: SEQ ID NO: 308:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   tggtgtaaac ttgtaccagt 20
<212> Type : DNA
<211> Length : 20
   SequenceName : SEQ ID NO: 309
   SequenceDescription :

### Feature

Sequence: SEQ ID NO: 309:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   acccatgaat ttgatggaga 20
<212> Type : DNA
<211> Length : 20
   SequenceName : SEQ ID NO: 310
   SequenceDescription :

### Feature

Sequence: SEQ ID NO: 310:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   ggaacctcat tggtagcagc 20
<212> Type : DNA
<211> Length : 20
   SequenceName : SEQ ID NO: 311
   SequenceDescription :

### Feature

Sequence: SEQ ID NO: 311:
<221> FeatureKey : primer_bind
<222> LocationFrom : 1
<222> LocationTo : 20
   Other Information :
   CDSJoin : No

### Sequence

<213> OrganismName : Artificial Sequence
<400> PreSequenceString :
   acttggaaaa cactgtgacc 20
<212> Type : DNA
<211> Length : 20
   SequenceName : SEQ ID NO: 312
   SequenceDescription :

### Feature

Sequence: SEQ ID NO: 312:
<221> FeatureKey : primer_bind
<222> LocationFrom : 1
<222> LocationTo : 20
   Other Information :
   CDSJoin : No

## Claims

1. A method for producing an immune status profile for a human and/or animal, the method comprising
(a) amplifying, in a first multiplex PCR amplification using nested target-specific primers, multiple DNAs and/or RNAs from a sample of white blood cells from a human or animal subject to produce multiple first amplicons, at least a portion of the inner target-specific primers comprising additional nucleotides to incorporate into the first amplicons a binding site for a common primer;
(b) separating the amplicons from the target-specific primers or diluting the amplicon/target specific primer mix so that there are significantly more amplicons than target-specific primers from the first amplification;
(c) amplifying, in a second amplification using at least one common primer, the first amplicons to produce multiple second amplicons; and
(d) sequencing the second amplicons to identify and quantify DNA sequences representing rearrangements to create an immune status profile.

2. The method of claim 1 further comprising the steps of (e) inputting the DNA sequences into a database to provide data which may be stored on a computer, server, or other electronic storage device, (f) inputting a data set of identifying information and characteristics for an individual corresponding to the sequences as data which may be stored on a computer, server, or other electronic storage device, and (g) evaluating the data of step (e) and step (f) for one or more individuals to determine whether a correlation exists between the sequences and one or more characteristics of the individual corresponding to the sequences.

3. The method of claim 1 wherein the target-specific primers are chosen from among the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, SEQ ID NO: 129, SEQ ID NO: 130, SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO:138, SEQ ID NO: 139, SEQ ID NO: 140, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, SEQ ID NO: 162, SEQ ID NO: 163, SEQ ID NO: 164, SEQ ID NO: 165, SEQ ID NO: 166, SEQ ID NO: 167, SEQ ID NO: 168, SEQ ID NO: 169, SEQ ID NO: 170, SEQ ID NO: 171, SEQ ID NO: 172, SEQ ID NO: 173, SEQ ID NO: 174, SEQ ID NO: 175, SEQ ID NO: 176, SEQ ID NO: 177, SEQ ID NO: 178, SEQ ID NO: 179, SEQ ID NO: 180, SEQ ID NO: 181, SEQ ID NO: 182, SEQ ID NO: 183, SEQ ID NO: 184, SEQ ID NO: 185, SEQ ID NO: 186, SEQ ID NO: 187, SEQ ID NO: 188, SEQ ID NO: 189, SEQ ID NO: 190, SEQ ID NO: 191, SEQ ID NO: 192, SEQ ID NO: 193, SEQ ID NO: 194, SEQ ID NO: 195, SEQ ID NO: 196, SEQ ID NO: 197, SEQ ID NO: 198, SEQ ID NO: 199, SEQ ID NO: 200, SEQ ID NO: 201, SEQ ID NO: 202, SEQ ID NO: 203, SEQ ID NO: 204, SEQ ID NO: 205, SEQ ID NO: 206, SEQ ID NO: 207, SEQ ID NO: 208, SEQ ID NO: 209, SEQ ID NO: 210, SEQ ID NO: 211, SEQ ID NO: 212, SEQ ID NO: 213, SEQ ID NO: 214, SEQ ID NO: 215, SEQ ID NO: 216, SEQ ID NO: 217, SEQ ID NO: 218, SEQ ID NO: 219, SEQ ID NO: 220, SEQ ID NO: 221, SEQ ID NO: 222, SEQ ID NO: 223, SEQ ID NO: 224, SEQ ID NO: 225, SEQ ID NO: 226, SEQ ID NO: 227, SEQ ID NO: 228, SEQ ID NO: 229, SEQ ID NO: 230, SEQ ID NO: 231, SEQ ID NO: 232, SEQ ID NO: 233, SEQ ID NO: 234, SEQ ID NO: 235, SEQ ID NO: 236, SEQ ID NO: 237, SEQ ID NO: 238, SEQ ID NO: 239, SEQ ID NO: 240, SEQ ID NO: 241, SEQ ID NO: 242, SEQ ID NO: 243, SEQ ID NO: 244, SEQ ID NO: 245, SEQ ID NO: 246, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255, SEQ ID NO: 256, SEQ ID NO: 257, SEQ ID NO: 258, SEQ ID NO: 259, SEQ ID NO: 260, SEQ ID NO: 261, SEQ ID NO: 262, SEQ ID NO: 263, SEQ ID NO: 264, SEQ ID NO: 265, SEQ ID NO: 266, SEQ ID NO: 267, SEQ ID NO: 268, SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, SEQ ID NO: 281, SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289, SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, SEQ ID NO: 300, SEQ ID NO: 301, SEQ ID NO: 302, SEQ ID NO: 303, SEQ ID NO: 304, SEQ ID NO: 305, SEQ ID NO: 306, SEQ ID NO: 307, SEQ ID NO: 308, SEQ ID NO: 309, SEQ ID NO: 310, SEQ ID NO: 311 and SEQ ID NO: 312.

## Patentansprüche

1. Ein Verfahren zur Erzeugung eines Immunstatusprofils für einen Menschen und/oder ein Tier, das Verfahren aufweisend:
(a) Amplifizieren, in einer ersten Mehrfach-PCR-Amplifikation unter Verwendung verschachtelter, Target-spezifischer Primer, mehrerer DNAs und/oder RNAs aus einer Probe von weißen Blutzellen aus einem menschlichen oder tierischen Subjekt zum Erzeugen mehrerer erster Amplicons, wobei zumindest ein Teil der inneren, Target-spezifischen Primer zusätzliche Nukleotide aufweist zum Integrieren einer Bindungsstelle für einen üblichen Primer in die ersten Amplicons;
(b) Trennen der Amplicons von den Target-spezifischen Primern oder Verdünnen der Amplicon/Target-spezifischer Primer Mischung, so dass es signifikant mehr Amplicons als Targetspezifische Primer aus der ersten Amplifikation gibt;
(c) Amplifizieren, in einer zweiten Amplifikation unter Verwendung zumindest eines üblichen Primers, der ersten Amplicons zum Erzeugen mehrerer zweiter Amplicons; und
(d) Sequenzieren der zweiten Amplicons zum Identifizieren und Quantifizieren von DNA-Sequenzen, welche Umlagerungen repräsentieren, zum Erschaffen eines Immunstatusprofils.

2. Das Verfahren von Anspruch 1, weiter aufweisend die Schritte von (e) Eingeben der DNA-Sequenzen in eine Datenbank zum Bereitstellen von Daten, welche auf einem Computer, Server oder einem anderen elektronischen Speichergerät gespeichert werden können, (f) Eingeben eines Datensatzes von Identifizierungsinformation und Charakteristiken für ein Individuum entsprechend der Sequenzen als Daten, welche auf einem Computer, Server oder einem anderen elektronischen Speichergerät gespeichert werden können, und (g) Evaluieren der Daten von Schritt (e) und Schritt (f) für ein oder mehrere Individuen zum Bestimmen, ob eine Korrelation zwischen den Sequenzen und einer oder mehrerer Charakteristiken von dem Individuum entsprechend der Sequenzen besteht.

3. Das Verfahren von Anspruch 1, wobei die Target-spezifischen Primer ausgewählt werden unter der Gruppe bestehend aus Seq.-ID Nr. 1, Seq.-ID Nr. 2, Seq.-ID Nr. 3, Seq.-ID Nr. 4, Seq.-ID Nr. 5, Seq.-ID Nr. 6, Seq.-ID Nr. 7, Seq.-ID Nr. 8, Seq.-ID Nr. 9, Seq.-ID Nr. 10, Seq.-ID Nr. 11, Seq.-ID Nr. 12, Seq.-ID Nr. 13, Seq.-ID Nr. 14, Seq.-ID Nr. 15, Seq.-ID Nr. 16, Seq.-ID Nr. 17, Seq.-ID Nr. 18, Seq.-ID Nr. 19, Seq.-ID Nr. 20, Seq.-ID Nr. 31, Seq.-ID Nr. 32, Seq.-ID Nr. 33, Seq.-ID Nr. 34, Seq.-ID Nr. 35, Seq.-ID Nr. 36, Seq.-ID Nr. 37, Seq.-ID Nr. 38, Seq.-ID Nr. 39, Seq.-ID Nr. 40, Seq.-ID Nr. 41, Seq.-ID Nr. 42, Seq.-ID Nr. 43, Seq.-ID Nr. 44, Seq.-ID Nr. 45, Seq.-ID Nr. 46, Seq.-ID Nr. 47, Seq.-ID Nr. 48, Seq.-ID Nr. 49, Seq.-ID Nr. 50, Seq.-ID Nr. 51, Seq.-ID Nr. 52, Seq.-ID Nr. 53, Seq.-ID Nr. 54, Seq.-ID Nr. 55, Seq.-ID Nr. 56, Seq.-ID Nr. 57, Seq.-ID Nr. 58, Seq.-ID Nr. 59, Seq.-ID Nr. 60, Seq.-ID Nr. 61, Seq.-ID Nr. 62, Seq.-ID Nr. 63, Seq.-ID Nr. 64, Seq.-ID Nr. 65, Seq.-ID Nr. 66, Seq.-ID Nr. 67, Seq.-ID Nr. 68, Seq.-ID Nr. 69, Seq.-ID Nr. 70, Seq.-ID Nr. 71, Seq.-ID Nr. 72, Seq.-ID Nr. 73, Seq.-ID Nr. 74, Seq.-ID Nr. 75, Seq.-ID Nr. 76, Seq.-ID Nr. 77, Seq.-ID Nr. 78, Seq.-ID Nr. 79, Seq.-ID Nr. 80, Seq.-ID Nr. 81, Seq.-ID Nr. 82, Seq.-ID Nr. 83, Seq.-ID Nr. 84, Seq.-ID Nr. 85, Seq.-ID Nr. 86, Seq.-ID Nr. 87, Seq.-ID Nr. 88, Seq.-ID Nr. 89, Seq.-ID Nr. 90, Seq.-ID Nr. 101, Seq.-ID Nr. 102, Seq.-ID Nr. 103, Seq.-ID Nr. 104, Seq.-ID Nr. 105, Seq.-ID Nr. 106, Seq.-ID Nr. 107, Seq.-ID Nr. 108, Seq.-ID Nr. 109, Seq.-ID Nr. 110, Seq.-ID Nr. 111, Seq.-ID Nr. 112, Seq.-ID Nr. 113, Seq.-ID Nr. 114, Seq.-ID Nr. 115, Seq.-ID Nr. 116, Seq.-ID Nr. 117, Seq.-ID Nr. 118, Seq.-ID Nr. 119, Seq.-ID Nr. 120, Seq.-ID Nr. 121, Seq.-ID Nr. 122, Seq.-ID Nr. 123, Seq.-ID Nr. 124, Seq.-ID Nr. 125, Seq.-ID Nr. 126, Seq.-ID Nr. 127, Seq.-ID Nr. 128, Seq.-ID Nr. 129, Seq.-ID Nr. 130, Seq.-ID Nr. 131, Seq.-ID Nr. 132, Seq.-ID Nr. 133, Seq.-ID Nr. 134, Seq.-ID Nr. 135, Seq.-ID Nr. 136, Seq.-ID Nr. 137, Seq.-ID Nr. 138, Seq.-ID Nr. 139, Seq.-ID Nr. 140, Seq.-ID Nr. 151, Seq.-ID Nr. 152, Seq.-ID Nr. 153, Seq.-ID Nr. 154, Seq.-ID Nr. 155, Seq.-ID Nr. 156, Seq.-ID Nr. 157, Seq.-ID Nr. 158, Seq.-ID Nr. 159, Seq.-ID Nr. 160, Seq.-ID Nr. 161, Seq.-ID Nr. 162, Seq.-ID Nr. 163, Seq.-ID Nr. 164, Seq.-ID Nr. 165, Seq.-ID Nr. 166, Seq.-ID Nr. 167, Seq.-ID Nr. 168, Seq.-ID Nr. 169, Seq.-ID Nr. 170, Seq.-ID Nr. 171, Seq.-ID Nr. 172, Seq.-ID Nr. 173, Seq.-ID Nr. 174, Seq.-ID Nr. 175, Seq.-ID Nr. 176, Seq.-ID Nr. 177, Seq.-ID Nr. 178, Seq.-ID Nr. 179, Seq.-ID Nr. 180, Seq.-ID Nr. 181, Seq.-ID Nr. 182, Seq.-ID Nr. 183, Seq.-ID Nr. 184, Seq.-ID Nr. 185, Seq.-ID Nr. 186, Seq.-ID Nr. 187, Seq.-ID Nr. 188, Seq.-ID Nr. 189, Seq.-ID Nr. 190, Seq.-ID Nr. 191, Seq.-ID Nr. 192, Seq.-ID Nr. 193, Seq.-ID Nr. 194, Seq.-ID Nr. 195, Seq.-ID Nr. 196, Seq.-ID Nr. 197, Seq.-ID Nr. 198, Seq.-ID Nr. 199, Seq.-ID Nr. 200, Seq.-ID Nr. 201, Seq.-ID Nr. 202, Seq.-ID Nr. 203, Seq.-ID Nr. 204, Seq.-ID Nr. 205, Seq.-ID Nr. 206, Seq.-ID Nr. 207, Seq.-ID Nr. 208, Seq.-ID Nr. 209, Seq.-ID Nr. 210, Seq.-ID Nr. 211, Seq.-ID Nr. 212, Seq.-ID Nr. 213, Seq.-ID Nr. 214, Seq.-ID Nr. 215, Seq.-ID Nr. 216, Seq.-ID Nr. 217, Seq.-ID Nr. 218, Seq.-ID Nr. 219, Seq.-ID Nr. 220, Seq.-ID Nr. 221, Seq.-ID Nr. 222, Seq.-ID Nr. 223, Seq.-ID Nr. 224, Seq.-ID Nr. 225, Seq.-ID Nr. 226, Seq.-ID Nr. 227, Seq.-ID Nr. 228, Seq.-ID Nr. 229, Seq.-ID Nr. 230, Seq.-ID Nr. 231, Seq.-ID Nr. 232, Seq.-ID Nr. 233, Seq.-ID Nr. 234, Seq.-ID Nr. 235, Seq.-ID Nr. 236, Seq.-ID Nr. 237, Seq.-ID Nr. 238, Seq.-ID Nr. 239, Seq.-ID Nr. 240, Seq.-ID Nr. 241, Seq.-ID Nr. 242, Seq.-ID Nr. 243, Seq.-ID Nr. 244, Seq.-ID Nr. 245, Seq.-ID Nr. 246, Seq.-ID Nr. 247, Seq.-ID Nr. 248, Seq.-ID Nr. 249, Seq.-ID Nr. 250, Seq.-ID Nr. 251, Seq.-ID Nr. 252, Seq.-ID Nr. 253, Seq.-ID Nr. 254, Seq.-ID Nr. 255, Seq.-ID Nr. 256, Seq.-ID Nr. 257, Seq.-ID Nr. 258, Seq.-ID Nr. 259, Seq.-ID Nr. 260, Seq.-ID Nr. 261, Seq.-ID Nr. 262, Seq.-ID Nr. 263, Seq.-ID Nr. 264, Seq.-ID Nr. 265, Seq.-ID Nr. 266, Seq.-ID Nr. 267, Seq.-ID Nr. 268, Seq.-ID Nr. 269, Seq.-ID Nr. 270, Seq.-ID Nr. 271, Seq.-ID Nr. 272, Seq.-ID Nr. 273, Seq.-ID Nr. 274, Seq.-ID Nr. 275, Seq.-ID Nr. 276, Seq.-ID Nr. 277, Seq.-ID Nr. 278, Seq.-ID Nr. 279, Seq.-ID Nr. 280, Seq.-ID Nr. 281, Seq.-ID Nr. 282, Seq.-ID Nr. 283, Seq.-ID Nr. 284, Seq.-ID Nr. 285, Seq.-ID Nr. 286, Seq.-ID Nr. 287, Seq.-ID Nr. 288, Seq.-ID Nr. 289, Seq.-ID Nr. 290, Seq.-ID Nr. 291, Seq.-ID Nr. 292, Seq.-ID Nr. 293, Seq.-ID Nr. 294, Seq.-ID Nr. 295, Seq.-ID Nr. 296, Seq.-ID Nr. 297, Seq.-ID Nr. 298, Seq.-ID Nr. 299, Seq.-ID Nr. 300, Seq.-ID Nr. 301, Seq.-ID Nr. 302, Seq.-ID Nr. 303, Seq.-ID Nr. 304, Seq.-ID Nr. 305, Seq.-ID Nr. 306, Seq.-ID Nr. 307, Seq.-ID Nr. 308, Seq.-ID Nr. 309, Seq.-ID Nr. 310, Seq.-ID Nr. 311 und Seq.-ID Nr. 312.

## Revendications

1. Procédé de production d'un profil de statut immunologique pour un humain et/ou un animal, le procédé comprenant les étapes consistant à :
(a) amplifier, dans une première amplification PCR multiplex utilisant des amorces spécifiques de la cible imbriquées, de multiples ADN et/ou ARN venant d'un échantillon de globules blancs d'un humain ou d'un animal susceptible de produire de multiples premiers amplicons, au moins une partie des amorces spécifiques de la cible internes comprenant des nucléotides supplémentaires pour incorporer aux premiers amplicons un site de liaison pour une amorce commune ;
(b) séparer les amplicons des amorces spécifiques de la cible ou diluer le mélange d'amplicons et d'amorces spécifiques de la cible de sorte qu'il y ait notablement plus d'amplicons que d'amorces spécifiques de la cible provenant de la première amplification ;
(c) amplifier, dans une seconde amplification utilisant au moins une amorce commune, les premiers amplicons pour produire de multiples seconds amplicons ; et
(d) séquencer les seconds amplicons pour identifier et quantifier des séquences d'ADN représentant des réaménagements afin de créer un profil de statut immunologique.

2. Procédé selon la revendication 1, comprenant en outre les étapes consistant à : (e) saisir les séquences d'ADN dans une base de données pour fournir des données qui peuvent être stockées sur un ordinateur, un serveur ou un autre dispositif de stockage électronique, f) saisir un jeu de données d'informations d'identification et de caractéristiques pour un individu correspondant aux séquences comme données qui peuvent être stockées sur un ordinateur, un serveur ou un autre dispositif de stockage électronique, et g) évaluer les données de l'étape (e) et de l'étape (f) pour un ou plusieurs individus afin de déterminer si une corrélation existe entre les séquences et une ou plusieurs caractéristiques de l'individu correspondant aux séquences.

3. Procédé selon la revendication 1, dans lequel les amorces spécifiques de la cible sont choisies dans le groupe constitué des suivantes :
SEQ ID n° 1, SEQ ID n° 2, SEQ ID n° 3, SEQ ID n° 4, SEQ ID n° 5, SEQ ID n° 6, SEQ ID n° 7, SEQ ID n° 8, SEQ ID n° 9, SEQ ID n° 10, SEQ ID n° 11, SEQ ID n° 12, SEQ ID n° 13, SEQ ID n° 14, SEQ ID n° 15, SEQ ID n° 16, SEQ ID n° 17, SEQ ID n° 18, SEQ ID n° 19, SEQ ID n° 20, SEQ ID n° 31, SEQ ID n° 32, SEQ ID n° 33, SEQ ID n° 34, SEQ ID n° 35, SEQ ID n° 36, SEQ ID n° 37, SEQ ID n° 38, SEQ ID n° 39, SEQ ID n° 40, SEQ ID n° 41, SEQ ID n° 42, SEQ ID n° 43, SEQ ID n° 44, SEQ ID n° 45, SEQ ID n° 46, SEQ ID n° 47, SEQ ID n° 48, SEQ ID n° 49, SEQ ID n° 50, SEQ ID n° 51, SEQ ID n° 52, SEQ ID n° 53, SEQ ID n° 54, SEQ ID n° 55, SEQ ID n° 56, SEQ ID n° 57, SEQ ID n° 58, SEQ ID n° 59, SEQ ID n° 60, SEQ ID n° 61, SEQ ID n° 62, SEQ ID n° 63, SEQ ID n° 64, SEQ ID n° 65, SEQ ID n° 66, SEQ ID n° 67, SEQ ID n° 68, SEQ ID n° 69, SEQ ID n° 70, SEQ ID n° 71, SEQ ID n° 72, SEQ ID n° 73, SEQ ID n° 74, SEQ ID n° 75, SEQ ID n° 76, SEQ ID n° 77, SEQ ID n° 78, SEQ ID n° 79, SEQ ID n° 80, SEQ ID n° 81, SEQ ID n° 82, SEQ ID n° 83, SEQ ID n° 84, SEQ ID n° 85, SEQ ID n° 86, SEQ ID n° 87, SEQ ID n° 88, SEQ ID n° 89, SEQ ID n° 90, SEQ ID n° 101, SEQ ID n° 102, SEQ ID n° 103, SEQ ID n° 104, SEQ ID n° 105, SEQ ID n° 106, SEQ ID n° 107, SEQ ID n° 108, SEQ ID n° 109, SEQ ID n° 110, SEQ ID n° 111, SEQ ID n° 112, SEQ ID n° 113, SEQ ID n° 114, SEQ ID n° 115, SEQ ID n° 116, SEQ ID n° 117, SEQ ID n° 118, SEQ ID n° 119, SEQ ID n° 120, SEQ ID n° 121, SEQ ID n° 122, SEQ ID n° 123, SEQ ID n° 124, SEQ ID n° 125, SEQ ID n° 126, SEQ ID n° 127, SEQ ID n° 128, SEQ ID n° 129, SEQ ID n° 130, SEQ ID n° 131, SEQ ID n° 132, SEQ ID n° 133, SEQ ID n° 134, SEQ ID n° 135, SEQ ID n° 136, SEQ ID n° 137, SEQ ID n° 138, SEQ ID n° 139, SEQ ID n° 140, SEQ ID n° 151, SEQ ID n° 152, SEQ ID n° 153, SEQ ID n° 154, SEQ ID n° 155, SEQ ID n° 156, SEQ ID n° 157, SEQ ID n° 158, SEQ ID n° 159, SEQ ID n° 160, SEQ ID n° 161, SEQ ID n° 162, SEQ ID n° 163, SEQ ID n° 164, SEQ ID n° 165, SEQ ID n° 166, SEQ ID n° 167, SEQ ID n° 168, SEQ ID n° 169, SEQ ID n° 170, SEQ ID n° 171, SEQ ID n° 172, SEQ ID n° 173, SEQ ID n° 174, SEQ ID n° 175, SEQ ID n° 176, SEQ ID n° 177, SEQ ID n° 178, SEQ ID n° 179, SEQ ID n° 180, SEQ ID n° 181, SEQ ID n° 182, SEQ ID n° 183, SEQ ID n° 184, SEQ ID n° 185, SEQ ID n° 186, SEQ ID n° 187, SEQ ID n° 188, SEQ ID n° 189, SEQ ID n° 190, SEQ ID n° 191, SEQ ID n° 192, SEQ ID n° 193, SEQ ID n° 194, SEQ ID n° 195, SEQ ID n° 196, SEQ ID n° 197, SEQ ID n° 198, SEQ ID n° 199, SEQ ID n° 200, SEQ ID n° 201, SEQ ID n° 202, SEQ ID n° 203, SEQ ID n° 204, SEQ ID n° 205, SEQ ID n° 206, SEQ ID n° 207, SEQ ID n° 208, SEQ ID n° 209, SEQ ID n° 210, SEQ ID n° 211, SEQ ID n° 212, SEQ ID n° 213, SEQ ID n° 214, SEQ ID n° 215, SEQ ID n° 216, SEQ ID n° 217, SEQ ID n° 218, SEQ ID n° 219, SEQ ID n° 220, SEQ ID n° 221, SEQ ID n° 222, SEQ ID n° 223, SEQ ID n° 224, SEQ ID n° 225, SEQ ID n° 226, SEQ ID n° 227, SEQ ID n° 228, SEQ ID n° 229, SEQ ID n° 230, SEQ ID n° 232, SEQ ID n° 232 SEQ ID n° 234, SEQ ID n° 235, SEQ ID n° 236, SEQ ID n° 237, SEQ ID n° 238, SEQ ID n° 239, SEQ ID n° 240, SEQ ID n° 241, SEQ ID n° 242, SEQ ID n° 243, SEQ ID n° 244, SEQ ID n° 245, SEQ ID n° 246, SEQ ID n° 247, SEQ ID n° 248, SEQ ID n° 249, SEQ ID n° 250, SEQ ID n° 251, SEQ ID n° 252, SEQ ID n° 253, SEQ ID n° 254, SEQ ID n° 255, SEQ ID n° 256, SEQ ID n° 257, SEQ ID n° 258, SEQ ID n° 259, SEQ ID n° 260, SEQ ID n° 261, SEQ ID n° 262, SEQ ID n° 263, SEQ ID n° 264, SEQ ID n° 265, SEQ ID n° 266, SEQ ID n° 267, SEQ ID n° 268, SEQ ID n° 269, SEQ ID n° 270, SEQ ID n° 271, SEQ ID n° 272, SEQ ID n° 273, SEQ ID n° 274, SEQ ID n° 275, SEQ ID n° 276, SEQ ID n° 277, SEQ ID n° 278, SEQ ID n° 279, SEQ ID n° 280, SEQ ID n° 281, SEQ ID n° 282, SEQ ID n° 283, SEQ ID n° 284, SEQ ID n° 285, SEQ ID n° 286, SEQ ID n° 287, SEQ ID n° 288, SEQ ID n° 289, SEQ ID n° 290, SEQ ID n° 291, SEQ ID n° 292, SEQ ID n° 293, SEQ ID n° 294, SEQ ID n° 295, SEQ ID n° 296, SEQ ID n° 297, SEQ ID n° 298, SEQ ID n° 299, SEQ ID n° 300, SEQ ID n° 301, SEQ ID n° 302, SEQ ID n° 303, SEQ ID n° 304, SEQ ID n° 305, SEQ ID n° 306, SEQ ID n° 307, SEQ ID n° 308, SEQ ID n° 309, SEQ ID n° 310, SEQ ID n° 311 et SEQ ID n° 312.
